# EUROPEAN PATENT SPECIFICATION

(11) **EP 3 447 719 B1**
(45) Date of publication and mention of the grant of the patent: **03.01.2024**
(21) Application number: 17785799.2
(22) Date of filing: 05.04.2017
(51) Int. Cl.: G16H 20/13, A61J 3/00

(54) **PACKAGED SINGLE-DOSE-MEDICAMENT AUDIT SYSTEM, PACKAGED SINGLE-DOSE-MEDICAMENT INFORMATION OUTPUT METHOD, AND COMPUTER-READABLE MEDIUM**
SYSTEM ZUM AUDIT VERPACKTER EINZELDOSISMEDIKAMENTE, VERFAHREN ZUR AUSGABE VON INFORMATIONEN ÜBER VERPACKTE EINZELDOSISMEDIKAMENTE UND COMPUTER-LESBARES MEDIUM
SYSTÈME D'AUDIT DE MÉDICAMENT À DOSE UNIQUE CONDITIONNÉ, PROCÉDÉ DE SORTIE D'INFORMATIONS DE MÉDICAMENT À DOSE UNIQUE CONDITIONNÉ, ET SUPPORT LISIBLE PAR ORDINATEUR

(30) Priority: 21.04.2016 JP 2016085540
(43) Date of publication of application: 27.02.2019
(73) Proprietor: FUJIFILM Corporation, Tokyo 106-8620 (JP)
(72) Inventor: YONAHA, Makoto, Ashigarakami-gun Kanagawa 258-8538 (JP); OKABE, Yuki, Ashigarakami-gun Kanagawa 258-8538 (JP)
(74) Representative: Klunker IP Patentanwälte PartG mbB
(86) International application number: PCT/JP2017/014260
(87) International publication number: WO 2017/183457

(56) References cited:
- WO-A1-2014/050486
- JP-A- H09 253 164
- JP-A- 2012 208 862
- JP-A- 2014 064 836
- JP-A- 2015 095 124
- JP-A- 2015 095 124
- JP-A- 2016 015 093
- JP-A- 2016 031 676
- JP-A- 2016 031 676
- US-A1- 2009 012 820
- US-A1- 2012 101 847
- US-A1- 2015 178 674
- US-B1- 6 330 351

## Description

### BACKGROUND OF THE INVENTION

### 1. Field of the Invention

The present invention relates to a one-packaging medicine audit system, a method of outputting one-packaging medicine information, and a one-packaging medicine computer-readable recording medium.

### 2. Description of the Related Art

A medicine notebook is used in which information necessary for medical personnel is described, such as a dose history of a medicine for each patient. In recent years, digitization of the medicine notebook is advanced. For example, it is possible to use a medicine notebook application that can be installed in a mobile terminal apparatus.

The medicine notebook application installed in the mobile terminal apparatus is used to realize incorporation of information on a medicine represented in a two-dimensional code printed on a prescription into the medicine notebook application.

JP2007-007286A discloses a browsing system of a prescribed medicine. The mobile terminal apparatus is used in the browsing system disclosed in JP2007-007286A. The browsing system disclosed in JP2007-007286A has a browsing database and an application of a website capable of communicating with the mobile terminal apparatus.

In a case where data printed on the two-dimensional code is sent from the mobile terminal apparatus, the application of the website extracts medicine information that matches an extraction condition from the browsing database with a drug code included in the data sent from the mobile terminal apparatus as the extraction condition.

The application of the website displays the extracted medicine information using a browsing function of the mobile terminal apparatus. A term of the mobile terminal apparatus in the specification corresponds to a mobile phone in JP2007-007286A. A term of the medicine in the specification corresponds to a term of a drug in JP2007-007286A.

JP2012-203810A discloses a dispensing management apparatus that performs dispensing processing and a printing control such as a medicine notebook label based on a patient code in which personal dispensing information on a patient, such as one-packaging of the medicine, crushing of the medicine, or a printing size of a form, is recorded.

JP2015-118444A discloses a medicine management apparatus in which information relating to a dispensing pharmacy or a dose of the medicines dispensed in a medical institution and an image of the medicines obtained by imaging the medicines are stored in association with each other.

The medicine management apparatus disclosed in JP2015-118444A sends a dose notification to the mobile terminal apparatus owned by the patient. The dose notification is displayed on a display unit of the mobile terminal apparatus that receives the dose notification. A display screen of the dose notification displays a dose date, a dose time, a pack image of the medicines to be dosed, or the like.

In recent years, one-packaging dispensing is performed in which a plurality of types of medicines for one-time dose are packaged in a one-packaging bag. In the one-packaging dispensing, automatic processing by using a one-packaging processing apparatus is performed. In the one-packaging dispensing, a one-packaging medicine audit that inspects whether the plurality of medicines are correctly packaged in a one-packaging is implemented.

JP2015-002917A discloses a one-packaging medicine audit apparatus that determines whether medicine type of the medicine to be put into the one-packaging processing apparatus is correct or incorrect based on the dispensing information and medicine type information representing the type of the medicine to be put into the one-packaging processing apparatus.

A term of the one-packaging processing apparatus in the specification corresponds to a dividedly packaging machine in JP2015-002917A. A term of the one-packaging medicine audit in the specification corresponds to a dispensing audit in JP2015-002917A.

### SUMMARY OF THE INVENTION

The medicine notebook application installed in the mobile terminal apparatus acquires the information on the medicine using a function of the mobile terminal apparatus. For example, a reading apparatus of the mobile terminal apparatus is used, the two-dimensional code that is printed on the prescription or the like issued by a doctor and represents the information on the medicine described on the prescription is read, and thus the medicine notebook application can acquire the information on the medicine described on the prescription.

However, in a case where the two-dimensional code is not printed on the prescription or the like, the medicine notebook application cannot acquire the information on the medicine described on the prescription.

There is no description relating to the one-packaging dispensing in which the plurality of medicines to be dosed at one time are packaged in the one-packaging bag in JP2007-007286A. The browsing system disclosed in JP2007-007286A is not compatible with the one-packaging dispensing and the one-packaging medicine audit.

The dispensing management apparatus disclosed in JP2012-203810A can print the medicine notebook label and the medicine bag, but is not configured to be capable of outputting the information on the medicine having a format capable of being acquired by the medicine notebook application installed in the mobile terminal apparatus.

The mobile terminal apparatus that receives the notification from the medicine management apparatus disclosed in JP2015-118444A can browse the dose notification using the browsing function, and the medicine management apparatus disclosed in JP2015-118444A does not output the medicine information having the format capable of being acquired by the medicine notebook application installed in the mobile terminal apparatus.

There is no description relating to the medicine notebook application installed in the mobile terminal apparatus in JP2015-002917A, and a one-packaging medicine audit system disclosed in JP2015-002917A is not compatible with the medicine notebook application installed in the mobile terminal apparatus.

The invention is made in view of such circumstances, and a purpose of the invention is to provide a one-packaging medicine audit system, a method of outputting one-packaging medicine information, and a one-packaging medicine computer-readable recording medium.

The invention is defined in the independent claims.

The one-packaging medicine information covers information relating to the medicine described on a prescription and can employ a type, a name, or a model of the medicine. The one-packaging medicine information can employ information relating to the dose of the medicines subjected to the one-packaging processing.

Examples of the information relating to the dose of the medicines are a dose timing, a quantity dosed at one time, and a precaution in the case of the dose. Furthermore, the one-packaging medicine information may employ each image of a plurality of medicines one-packaged or an image of the medicines in the one-packaging bag.

The first output unit may be configured that one-packaging information having a different format can be output. For example, a configuration corresponding to each of data communication, printing, and display may be included.
It may be configured that the first output unit outputs the one-packaging medicine information as information having a format capable of being employed in a medicine notebook application executed by the mobile terminal apparatus.

Accordingly, it is possible to use the one-packaging information using the medicine notebook application executed by the mobile terminal apparatus.

It may further be configured that the first output unit encodes the one-packaging medicine information and outputs the encoded information.

Accordingly, it is possible to read the one-packaging medicine information by using a reading apparatus of the mobile terminal apparatus.

The one-packaging medicine information may be output by performing a one-dimensional encoding or a two-dimensional encoding.

It may further be configured that the first output unit outputs the one-packaging medicine information for medicines of which an audit determination is passed.

It is further possible to acquire the one-packaging medicine information of which the audit determination is passed in the mobile terminal apparatus.

Since contents of actually prescribed medicines are correctly reflected in the one-packaging medicine information of which the audit determination is passed, it is possible to use appropriate one-packaging medicine information.

It may further be configured that the first output unit outputs identification information on the medicines actually one-packaged by the one-packaging processing apparatus as the one-packaging medicine information.

Accordingly, even in a case where the medicine described on the prescription in the one-packaging processing is prescribed and replaced with a medicine having the same active ingredient, it is possible to acquire the identification information on the medicines actually one-packaged in the mobile terminal apparatus.

An example of the medicine having the same active ingredient is a follow-on drug or a medicine having the same active ingredient as an original drug.

An example of the identification information on the medicines is a medicine type representing a type of a medicine. Any identification information on the medicines may be employed as long as the plurality of medicines subjected to the one-packaging processing can be identified.

It may further be configured to further comprise an imaging data acquisition unit that acquires imaging data of medicines actually to be subjected to one-packaging processing by the one-packaging processing apparatus, and a second output unit that performs the output of the imaging data acquired by using the imaging data acquisition unit and the identification information on the medicines represented by the imaging data acquired by using the imaging data acquisition unit in correlation with each other.

Accordingly, it is possible to store the imaging data of the medicines actually one-packaged and the identification information on the medicines which is the one-packaging medicine information in correlation with each other.

It is possible to use the image of the medicines corresponding to the identification information on the medicines with the identification information on the medicines as key information.

Examples of the mobile terminal apparatus are a mobile phone and a portable plate computer. Examples of the one-packaging medicine information acquisition unit are the reading apparatus of the mobile phone, and a data communication terminal or a data communication unit. Examples of the reading apparatus are a camera and a code reader.

In the mobile terminal apparatus, it is possible to use the imaging data of the medicine that is stored in the imaging data storage apparatus and is correlated with the identification information on the medicines with reference to the identification information on the medicines.

It may be further configured that the mobile terminal apparatus further includes a display unit that displays an image for each one-packaging bag subjected to the one-packaging processing using the imaging data acquired by using the mobile terminal imaging data acquisition unit.

Accordingly, it is possible to display an image represented by the imaging data of the medicines for each one-packaging bag on the display unit.

The image for each one-packaging bag may be imaged before the medicines are put into the one-packaging bag. The image for each one-packaging bag may be imaged after the medicines are put into the one-packaging bag.

It may further be configured that the one-packaging medicine audit apparatus outputs dose information on medicines actually subjected to one-packaging processing by using the one-packaging processing apparatus as the one-packaging medicine information by using the first output unit, and the mobile terminal apparatus acquires the dose information on the medicines actually subjected to the one-packaging processing by using the one-packaging processing apparatus as the one-packaging medicine information by using the one-packaging medicine information acquisition unit, and displays an image represented by imaging data of medicines required to be dosed next time.

Accordingly, it is possible to grasp the medicines required to be dosed next time from the image of the medicines displayed on the display unit.

The dose information on the medicines required to be dosed next time may be displayed by using the display unit.

Examples of the dose information are the types of the medicines to be dosed at one time and the number of medicines for each medicine.

It may further be configured that the mobile terminal apparatus displays at least any one of an image represented by imaging data of medicines dosed in the past or an image represented by imaging data of medicines required to be dosed in the future by using the display unit.

Accordingly, it is possible to grasp at least any one of the medicines already dosed or the medicines required to be dosed in the future from the image of the medicines displayed on the display unit.

Herein, the dose information on the medicines dosed in the past may be displayed by using the display unit. The dose information on the medicines required to be dosed in the future may be displayed by using the display unit.

It may further be configured that the mobile terminal apparatus enlarges and displays the image represented by the imaging data of the medicines required to be dosed next time by using the display unit.

Accordingly, it is easy to grasp the dose timing of the medicines required to be dosed next time. The medicines required to be dosed next time are medicines to be dosed first among medicines not yet dosed.

It may further be configured that the mobile terminal apparatus highlights and displays information relating to a dose timing of the medicines required to be dosed next time with respect to the image represented by the imaging data of the medicines required to be dosed next time by using the display unit.

Accordingly, it is easy to grasp the dose timing of the medicines required to be dosed next time.

It may further be configured that the mobile terminal apparatus displays information for urging the dose according to an elapsed period from the dose timing by using the display unit.

Accordingly, it is possible to grasp the dose timing from the information displayed on the display unit and to prevent dose forgetting.

It may further be configured that the mobile terminal apparatus further includes a replenishment medicine specification unit that specifies a type of a medicine required to be replenished using the one-packaging medicine information acquired by using the one-packaging medicine information acquisition unit and dose history information representing actually dosed medicines, and displays the type of the medicine required to be replenished specified by using the replenishment medicine specification unit by using the display unit.

Accordingly, it is possible to grasp the medicines required to be replenished from the information displayed on the display unit and to prevent replenishment forgetting.

With the invention, the one-packaging medicine information that is related to the medicines actually subjected to the one-packaging processing by using the one-packaging processing apparatus and is acquired by using the one-packaging medicine audit apparatus can be acquired by the mobile terminal apparatus.

### BRIEF DESCRIPTION OF THE DRAWINGS

Fig. 1 is an explanatory diagram showing a configuration of a one-packaging medicine audit system and a procedure for a medicine prescription.
Fig. 2 is a block diagram showing a schematic configuration of a one-packaging medicine audit apparatus.
Fig. 3 is a block diagram showing a configuration example of a first output unit.
Fig. 4 is a block diagram showing another configuration example of the first output unit.
Fig. 5 is a block diagram showing a configuration example of a second output unit.
Fig. 6 is an explanatory diagram of correct or incorrect determination processing by using a put-in medicine type correct or incorrect determination unit.
Fig. 7 is an explanatory diagram of the correct or incorrect determination processing by using an appearance-based number correct or incorrect determination unit.
Fig. 8 is an explanatory diagram of determination processing by using a mix-up determination possibility determination unit.
Fig. 9 is an explanatory diagram of a mix-up in medicines.
Fig. 10 is an explanatory diagram of an example of a warning display in a case where the appearance-based number correct or incorrect determination unit determines that it is incorrect.
Fig. 11 is an explanatory diagram of an example of a warning display in a case where the mix-up determination possibility determination unit determines that a determination of presence or absence of the mix-up is impossible.
Fig. 12 is a flowchart showing a procedure for a one-packaging medicine audit including a method of outputting one-packaging medicine information.
Fig. 13 is a flowchart showing a procedure for determination processing in a one-packaging correct or incorrect determination step.
Fig. 14 is an explanatory diagram showing an example of a display screen in a case where a basic function of the medicine notebook application is executed.
Fig. 15 is an explanatory diagram showing an example of a display screen in the case where the basic function of the medicine notebook application is executed.
Fig. 16 is an explanatory diagram showing an example of a display screen in the case where the basic function of the medicine notebook application is executed.
Fig. 17 is an explanatory diagram showing an example of a display screen in a case where an urging function of the medicine notebook application is executed.
Fig. 18 is an explanatory diagram showing an example of a display screen in the case where the urging function of the medicine notebook application is executed.
Fig. 19 is an explanatory diagram showing an example of a display screen in the case where the urging function of the medicine notebook application is executed.
Fig. 20 is an explanatory diagram showing an example of a display screen in a case where a medicine shortage notification of the medicine notebook application is executed.
Fig. 21 is an explanatory diagram showing an example of a display screen in the case where the medicine shortage notification of the medicine notebook application is executed.
Fig. 22 is an explanatory diagram showing an example of a display screen in the case where the medicine shortage notification of the medicine notebook application is executed.

### DESCRIPTION OF THE PREFERRED EMBODIMENTS

Hereinafter, preferable embodiments of the invention will be described according to accompanying drawings. In the specification, the same reference numeral is assigned to a previously described configuration, and a description thereof is omitted as appropriate.

### [Description of One-Packaging Medicine Audit System]

### <Description of Procedure of Medicine Prescription>

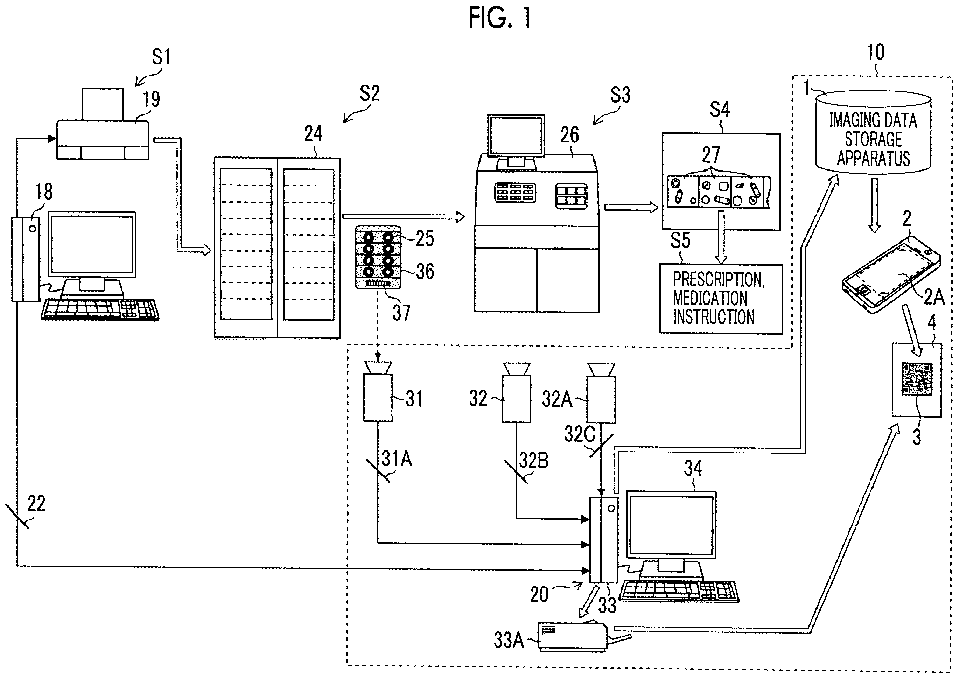

Fig. 1 is an explanatory diagram showing a configuration of a one-packaging medicine audit system and a procedure for a medicine prescription. The procedure for the medicine prescription shown in Fig. 1 includes a prescription input step S1, a picking step S2, an automatic one-packaging step S3, a one-packaging dispensing audit step S4, and a prescription step S5.

In the prescription input step S1, dispensing information described in a prescription is input to a receipt computer 18. An example of the dispensing information is an age or a name of a patient, a medicine type or a name of a medicine, a quantity of the medicine, usage of the medicine, or a dosage of the medicine. A term of the medicine type of the medicine in the specification is synonymous with a classification of the medicine or a type of the medicine. The dispensing information described on the prescription is one embodiment of prescription information.

Next, the dispensing information is printed from a printer 19 connected to the receipt computer 18. Dispensing information 22 is output from the receipt computer 18 to a one-packaging medicine audit apparatus 20.

In the picking step S2, a medicine 25 corresponding to the dispensing information 22 is picked from a medicine shelf 24 based on the dispensing information 22 described in a printed matter output from the printer 19. An example of the medicine 25 is a tablet or a capsule. In the picking step S2, for example, an automatic picking apparatus that automatically picks the medicine may be used based on the dispensing information 22 input to the receipt computer 18.

In the automatic one-packaging step S3, the medicines 25 picked in the picking step S2 are set for each amount to be packaged in a one-packaging bag 27 on a tray of a one-packaging processing apparatus 26. Then, the medicines 25 set on the tray are automatically one-packaged in a plurality of one-packaging bags 27 by using the one-packaging processing apparatus 26. Since the one-packaging processing apparatus 26 is publicly known, a specific description of a configuration thereof is omitted.

In the one-packaging dispensing audit step S4, it is confirmed whether the medicine types and the number of the medicines 25 one-packaged in the one-packaging bag 27 are correct by using the one-packaging medicine audit apparatus 20. That is, in the one-packaging dispensing audit step S4, a one-packaging the dispensing audit is performed in which it is confirmed whether the medicines one-packaged in the one-packaging bag 27 conforms to the dispensing information 22.

In the prescription step S5, the prescription of the medicines 25 one-packaged is performed. In the prescription step S5, a medication instruction for the patient is performed after the dispensing audit.

### <Description of One-Packaging Medicine Audit System>

The one-packaging medicine audit apparatus 20 and a mobile terminal apparatus 2 shown in Fig. 1 configure a one-packaging medicine information audit system 10. The one-packaging medicine information audit system 10 may include an imaging data storage apparatus 1.

The one-packaging medicine audit apparatus 20 shown in Fig. 1 includes a first camera 31, a second camera 32, a third camera 32A, a main body portion 33, a printer 33A, and a display unit 34. The first camera 31 images medicine type information 37 recorded on a package 36 of the picked medicines 25.

An example of the package 36 is a PTP sheet, a bottle, or a jar. The PTP is an abbreviation of press through package.

The medicine type information 37 is information indicating the medicine type such as a one-dimensional code, a character, or electronic identification information recorded in an RF tag or the like. In a case where the medicine type information 37 is the one-dimensional code, a one-dimensional code reader may be used as the first camera 31. The one-dimensional code is sometimes referred to as a barcode.

In a case where the medicine type information 37 is the electronic identification information stored in the RF tag, an RF tag reader may be used instead of the first camera 31. The RF is an abbreviation of radio frequency.

The second camera 32 images the medicines 25 to be packaged in each one-packaging bag 27 before the medicines 25 are packaged in the one-packaging bag 27. For example, the medicines 25 to be packaged in the one-packaging bag 27 set on the tray of the one-packaging processing apparatus 26 are imaged by using the second camera 32. In Fig. 1, the second camera 32 is disposed separately from the one-packaging processing apparatus 26. However, for example, the second camera 32 may be disposed inside the one-packaging processing apparatus 26.

The third camera 32A images the medicines 25 packaged in each one-packaging bag 27 after the medicines 25 are packaged in the one-packaging bag 27. In the imaging by using the third camera 32A, the medicines 25 inside the one-packaging bag 27 are imaged from a transparent surface side of the one-packaging bag 27.

First imaging data 31A imaged by using the first camera 31, second imaging data 32B imaged by using the second camera 32, and third imaging data 32C imaged by using the third camera 32A are sent to the main body portion 33.

A personal computer can be employed as the main body portion 33. The main body portion 33 is connected to the receipt computer 18, the first camera 31, the second camera 32, and the third camera 32A in a communicable manner.

The main body portion 33 determines whether the medicines 25 one-packaged in the one-packaging bag 27 are correct based on the dispensing information 22 input from the receipt computer 18, the first imaging data 31A, and the second imaging data 32B. The third imaging data 32C may be used instead of the second imaging data 32B. The second camera 32 is one embodiment of an imaging data acquisition unit. The third camera 32A is one embodiment of the imaging data acquisition unit. At least any one of the second camera 32 or the third camera 32A can be employed as the imaging data acquisition unit.

In a case where the medicines 25 one-packaged in the one-packaging bag 27 are determined to be incorrect, the main body portion 33 outputs a determination result indicating the fact to the display unit 34. In Fig. 1, the main body portion 33 is provided separately from the one-packaging processing apparatus 26, but a computer of the one-packaging processing apparatus 26 may be used as the main body portion 33. The computer of the one-packaging processing apparatus 26 includes a control unit of the one-packaging processing apparatus 26.

The printer 33A is an output apparatus of the one-packaging medicine information in the one-packaging medicine audit apparatus 20. The printer 33A prints a two-dimensional code 3 in which the one-packaging medicine information acquired by using the one-packaging medicine audit apparatus 20 is represented on a paper medium 4.

The one-packaging medicine information is one-packaging medicine information relating to the medicines actually one-packaged by using the one-packaging processing apparatus 26 and one-packaging medicine information acquired by using the one-packaging medicine audit apparatus 20. The one-packaging medicine information corresponds to the prescription information which is information relating to the prescription of the medicine described on the prescription and covers information relating to the prescription of the medicine included in the prescription information.

For example, a type, a name, or a model of the medicine can be employed as the one-packaging medicine information. Dose information which is information relating to the dose of the medicines subjected to one-packaging processing can be employed as the one-packaging medicine information. The dose information includes the identification information on the medicines dosed at one time and the quantity for each medicine.

The display unit 34 displays the determination result output from the main body portion 33. A monitor of the one-packaging processing apparatus 26 may be employed as the display unit 34. The display unit 34 can function as the output apparatus of the one-packaging medicine information in the one-packaging medicine audit apparatus 20. For example, the two-dimensional code 3 shown in Fig. 1 may be displayed.

In the imaging data storage apparatus 1 shown in Fig. 1, at least any one of the second imaging data 32B imaged by using the second camera 32 or the third imaging data 32C imaged by using the third camera 32A is stored in correlation with the one-packaging medicine information.

The second imaging data 32B is one embodiment of imaging data of the medicines subjected to the one-packaging processing. The third imaging data 32C is one embodiment of the imaging data of the medicines subjected to the one-packaging processing.

That is, the imaging data of one-packaging medicines stored in the imaging data storage apparatus 1 may be the imaging data of the medicines packaged in each one-packaging bag 27 after the medicines are packaged in the one-packaging bag 27 or may be the imaging data of the medicines to be packaged in the one-packaging bag 27 before the medicines are packaged in the one-packaging bag 27.

The medicine notebook application can be installed in the mobile terminal apparatus 2 shown in Fig. 1. The medicine notebook application is an application that can be executed in the mobile terminal apparatus 2 and an application that realizes the digitized function of the medicine notebook by using the mobile terminal apparatus 2.

In a case where the two-dimensional code 3 is read by using the mobile terminal apparatus 2, the one-packaging medicine information represented in the two-dimensional code 3 can be used by the medicine notebook application installed in the mobile terminal apparatus 2.

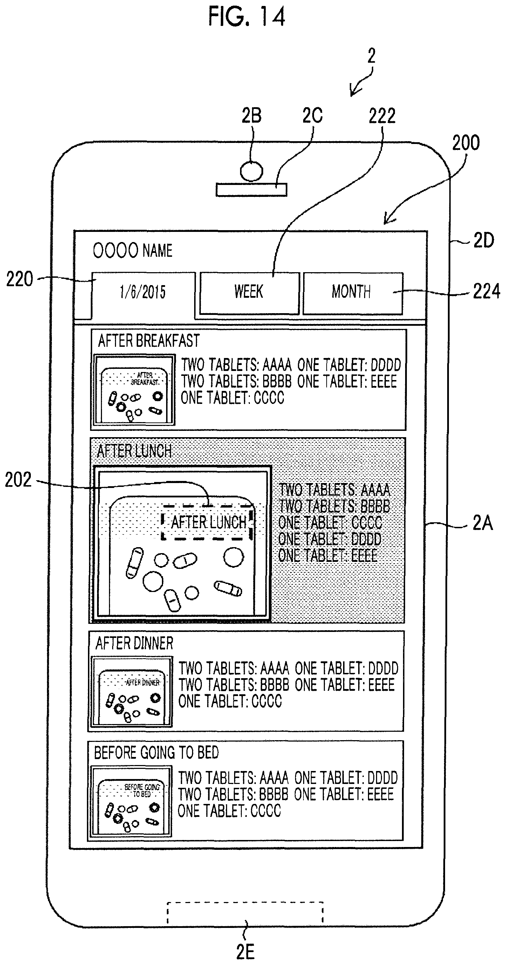

A camera included in the mobile terminal apparatus 2 can be employed for reading the two-dimensional code 3 by using the mobile terminal apparatus 2. The camera included in the mobile terminal apparatus 2 is illustrated by a reference numeral 2B in Figs. 14 to 22.

The mobile terminal apparatus 2 can acquire the imaging data of the one-packaging medicines stored in the imaging data storage apparatus 1 with the one-packaging medicine information that can be used by the medicine notebook application as key information.

That is, the mobile terminal apparatus 2 and the imaging data storage apparatus 1 are configured to be capable of data communication. The data communication may be a wireless format or a wired format. The data communication may be performed between the mobile terminal apparatus 2 and the imaging data storage apparatus 1 through a network.

The imaging data of the one-packaging medicines read by using the mobile terminal apparatus 2 can be displayed on a touch panel 2A of the mobile terminal apparatus using the function of the medicine notebook application. The detail of the medicine notebook application will be described below.

### [Configuration of One-packaging Medicine Audit Apparatus]

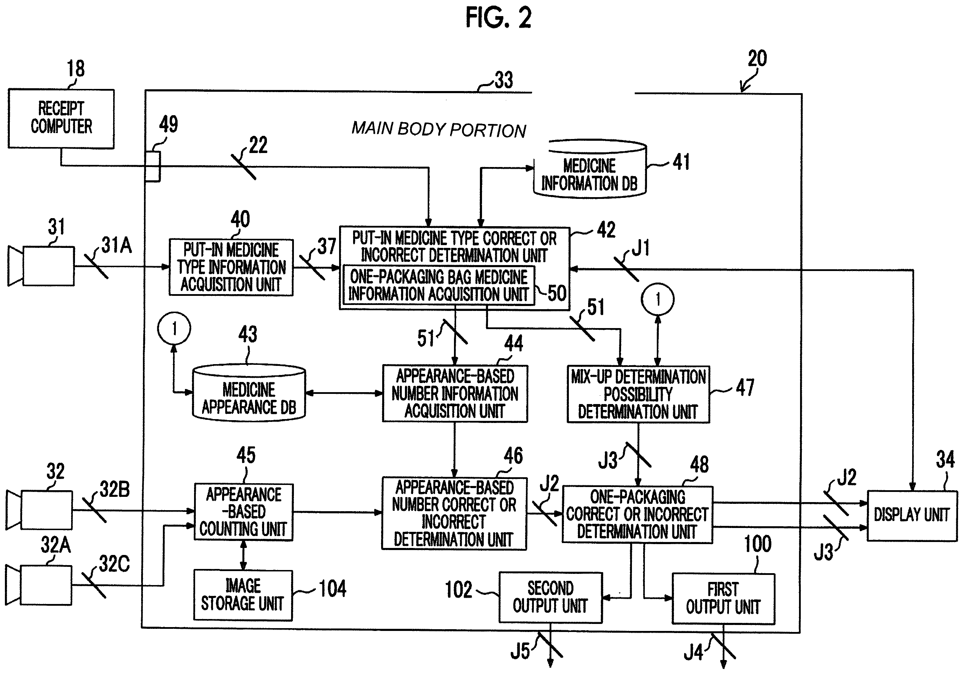

Fig. 2 is a block diagram showing a schematic configuration of the one-packaging medicine audit apparatus. The medicine 25 and the one-packaging bag 27 in the following description are shown in Fig. 1.

The main body portion 33 of the one-packaging medicine audit apparatus 20 shown in Fig. 2 comprises a put-in medicine type information acquisition unit 40, a medicine information database 41, a put-in medicine type correct or incorrect determination unit 42, a medicine appearance database 43, an appearance-based number information acquisition unit 44, an appearance-based counting unit 45, an appearance-based number correct or incorrect determination unit 46, a mix-up determination possibility determination unit 47, a one-packaging correct or incorrect determination unit 48, a communication interface 49, a first output unit 100, and a second output unit 102. In Fig. 2, the medicine information database is described as a medicine information DB. The medicine appearance database is described as a medicine appearance DB.

In the put-in medicine type information acquisition unit 40, the medicine type information 37 is extracted from the first imaging data 31A obtained by imaging by using the first camera 31. For example, in the put-in medicine type information acquisition unit 40, at least any one of the one-dimensional code or the character recorded on the package 36 is extracted.

In the put-in medicine type information acquisition unit 40, the medicine type information 37 is input to the put-in medicine type correct or incorrect determination unit 42. The put-in medicine type information acquisition unit 40 may be provided inside the first camera 31. The first imaging data 31A may be reading information on the one-dimensional code.

A correspondence relationship between the medicine type of the medicine 25 and the medicine type information 37 is stored in the medicine information database 41. A correspondence relationship between an original drug and a follow-on drug is stored in the medicine information database 41. The detail of the information stored in the medicine information database 41 will be described below.

In the put-in medicine type correct or incorrect determination unit 42, it is determined whether the medicine types of the medicines 25 to be put into the one-packaging processing apparatus 26 are correct with reference to the medicine information database 41 based on the dispensing information 22 input from the receipt computer 18 through the communication interface 49 and the medicine type information 37 input from the put-in medicine type information acquisition unit 40.

In the put-in medicine type correct or incorrect determination unit 42, in a case where the medicine types of the medicines 25 to be put into the one-packaging processing apparatus 26 shown in Fig. 1 are determined to be incorrect, a determination result J1 including information on the medicine type is output to the display unit 34.

Accordingly, the display unit 34 can display that the medicine types of the medicines 25 to be put into the one-packaging processing apparatus 26 are incorrect and the medicine type of the incorrect medicine.

The put-in medicine type correct or incorrect determination unit 42 comprises a one-packaging bag medicine information acquisition unit 50. In the one-packaging bag medicine information acquisition unit 50, one-packaging bag medicine information 51 indicating the medicine types of the medicines 25 to be one-packaged in the one-packaging bags 27 in the one-packaging processing apparatus 26 and the number of medicines for each medicine type is acquired with reference to the medicine information database 41 based on the dispensing information 22 and the medicine type information 37 of the picked medicine 25.

The one-packaging bag medicine information acquisition unit 50 outputs the one-packaging bag medicine information 51 to the appearance-based number information acquisition unit 44 and the mix-up determination possibility determination unit 47.

The medicine type of the medicine 25 and medicine appearance information indicating the appearance of the medicine are stored in association with each other in the medicine appearance database 43. The medicine appearance information includes information indicating a length of the medicine 25, a shape of the medicine 25, a color of the medicine 25, or the like. The detail of the information stored in the medicine appearance database 43 will be described below.

An example of the length of the medicine 25 is an area, a diameter, a radius, a length of a major axis, or a length of a minor axis. An example of the shape of the medicine 25 is a circle, a triangle, an ellipse, or a capsule shape. An example of the color of the medicine 25 is an RGB value. R of RGB represents red. G of RGB represents green. B of RGB represents blue.

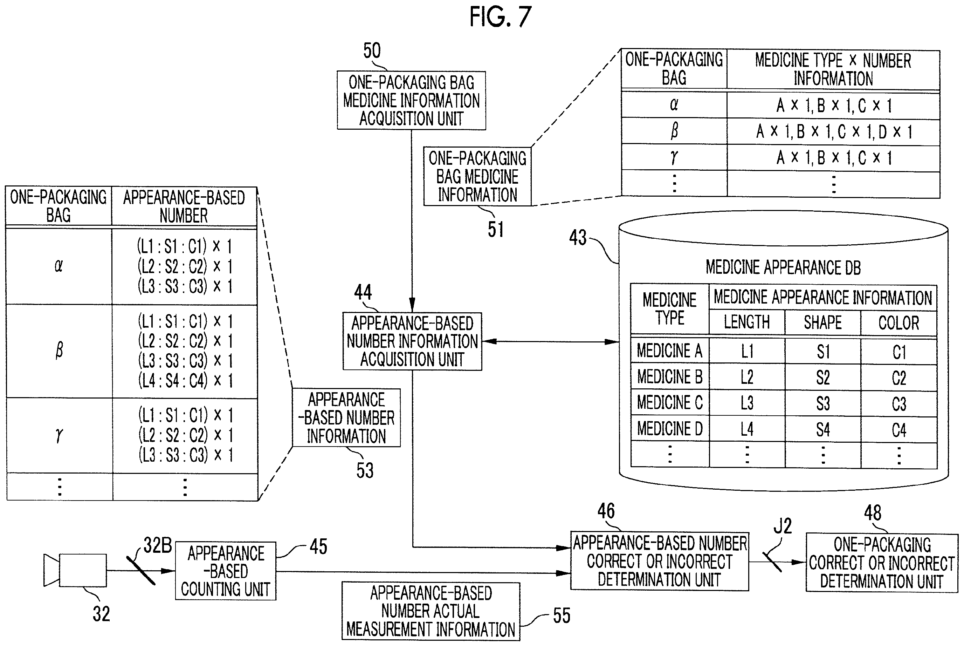

In the appearance-based number information acquisition unit 44, appearance-based number information indicating the appearance-based number which is the number of the medicines 25 to be one-packaged in the one-packaging bags 27 by the appearance is acquired with reference to the medicine appearance database 43 based on the one-packaging bag medicine information 51 input from the one-packaging bag medicine information acquisition unit 50. The appearance-based number information is illustrated by a reference numeral 53 in Fig. 7. The detail of the appearance-based number information will be described below.

In the appearance-based counting unit 45, image data of the medicines 25 to be packaged in the one-packaging bag 27 before the one-packaging which is represented in the second imaging data 32B is analyzed, and the appearance-based number of the medicines 25 to be one-packaged for each one-packaging bag 27 is counted.

Alternatively, in the appearance-based counting unit 45, image data of the medicines 25 one-packaged in the one-packaging bag 27 which is represented in the third imaging data 32C is analyzed, and the appearance-based number of the medicines 25 one-packaged for each one-packaging bag 27 is counted.

In the appearance-based number correct or incorrect determination unit 46, the appearance-based number information input from the appearance-based number information acquisition unit 44 is collated with actual measurement information input from the appearance-based counting unit 45. The actual measurement information is illustrated by a reference numeral 55 in Fig. 7.

In the appearance-based number correct or incorrect determination unit 46, it is determined whether the appearance-based number of the medicines 25 to be one-packaged in the one-packaging bags 27 is correct based on whether the actual measurement information matches the appearance-based number information. A determination result J2 is output to the one-packaging correct or incorrect determination unit 48.

In the appearance-based number correct or incorrect determination unit 46, in a case where the appearance-based number of the medicines 25 to be put into the one-packaging bags 27 is incorrect, the determination result J2 including information indicating the one-packaging bag 27 is output to the one-packaging correct or incorrect determination unit 48.

In the mix-up determination possibility determination unit 47, it is determined whether a determination of the presence or absence of the mix-up in the medicines 25 is possible with reference to the medicine appearance database 43 based on the one-packaging bag medicine information 51 input from the one-packaging bag medicine information acquisition unit 50. A determination result J3 is output to the one-packaging correct or incorrect determination unit 48.

In the one-packaging correct or incorrect determination unit 48, it is determined whether the medicines 25 one-packaged in the one-packaging bag 27 are correct based on the determination result J2 of the appearance-based number correct or incorrect determination unit 46 and the determination result J3 of the mix-up determination possibility determination unit 47.

In the one-packaging correct or incorrect determination unit 48, in a case where the appearance-based number of the medicines 25 to be put into the one-packaging bag 27 is correct and the determination of the presence or absence of the mix-up in the medicines 25 is possible, the medicines 25 one-packaged in the one-packaging bag 27 are determined to be correct.

On the other hand, in the one-packaging correct or incorrect determination unit 48, in a case where the appearance-based number of the medicines 25 to be one-packaged in the one-packaging bags 27 is incorrect, it is determined that there is an incorrection in the medicines 25 one-packaged in the one-packaging bag 27.

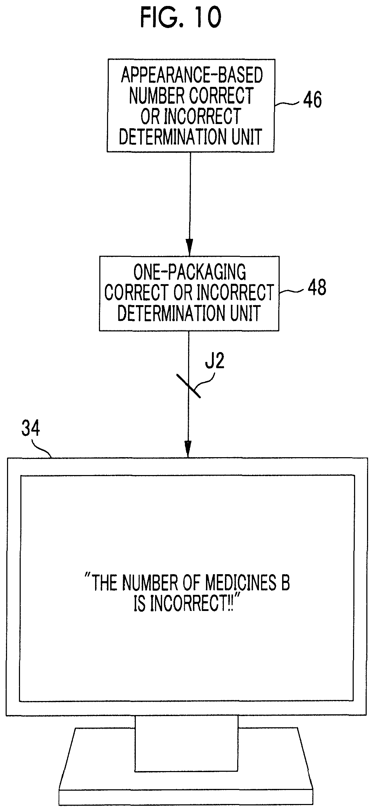

In the one-packaging correct or incorrect determination unit 48, the determination result J2 indicating the one-packaging bag 27 in which the appearance-based number is incorrect is output to the display unit 34. The display unit 34 can display warning information indicating the one-packaging bag 27 in which the appearance-based number is incorrect. A display example of the warning information indicating the one-packaging bag 27 in which the appearance-based number is incorrect is shown in Fig. 10.

In the one-packaging correct or incorrect determination unit 48, in a case where the determination of the presence or absence of the mix-up in different types of medicines 25 is impossible, it is determined that there is a possibility that the medicines 25 one-packaged in the one-packaging bag 27 are incorrect. The one-packaging correct or incorrect determination unit 48 outputs the determination result J3 indicating the medicine type of the medicine 25 of which the determination of the presence or absence of the mix-up is impossible to the display unit 34.

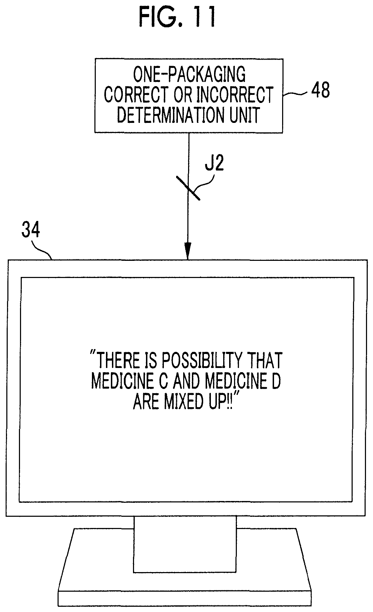

Accordingly, the display unit 34 displays warning information indicating the medicine type of the medicine 25 of which the determination of the presence or absence of the mix-up is impossible. A display example of the warning information indicating the medicine type of the medicine 25 of which the determination of the presence or absence of the mix-up is impossible is shown in Fig. 11.

In a case where the appearance-based number of the medicines 25 to be one-packaged in the one-packaging bags 27 is incorrect and the determination of the presence or absence of the mix-up in the different types of medicines 25 is impossible, both pieces of warning information shown in Figs. 10 and 11 are displayed in the display unit 34.

In the first output unit 100, one-packaging medicine information J4 that is related to the medicines 25 actually subjected to the one-packaging processing by using the one-packaging processing apparatus 26 shown in Fig. 1 and is acquired by using the one-packaging medicine audit apparatus 20 is output. The first output unit 100 corresponds to first output means.

In the second output unit 102, the second imaging data 32B which is the imaging data of the one-packaging medicines imaged and acquired by using the second camera 32 or the third imaging data 32C which is the imaging data of the one-packaging medicines imaged and acquired by using the third camera 32A is output in correlation with the one-packaging medicine information. The imaging data correlated with the one-packaging medicine information to be output from the second output unit 102 is illustrated by a reference numeral J5 in Fig. 2.

An image storage unit 104 stores at least any of the second imaging data 32B which is the imaging data of the one-packaging medicines imaged and acquired by using the second camera 32 or the third imaging data 32C which is the imaging data of the one-packaging medicines imaged and acquired by using the third camera 32A.

### [Description of Configuration Example of First Output Unit]

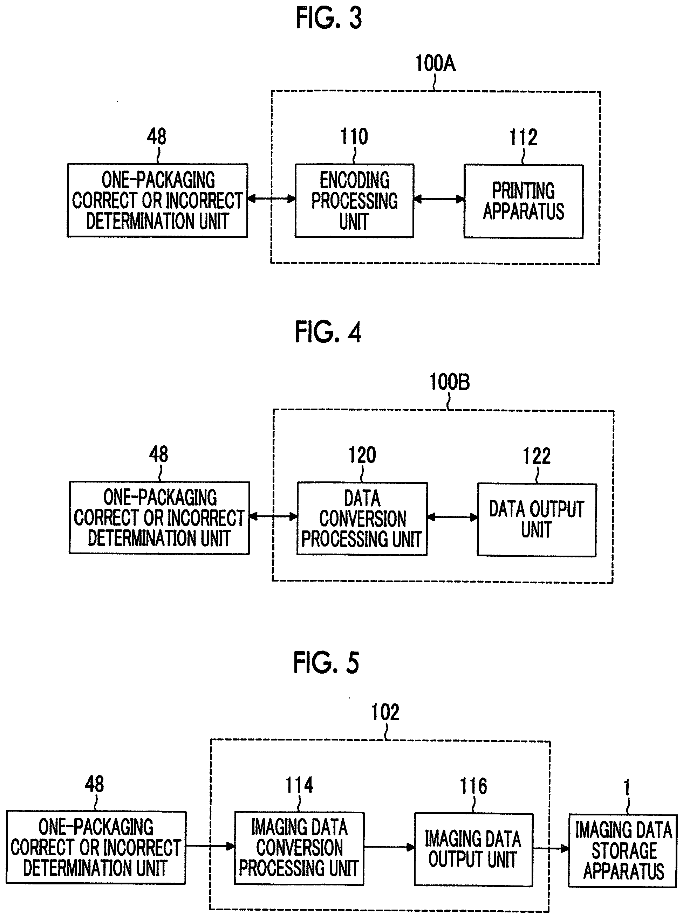

Fig. 3 is a block diagram showing a configuration example of the first output unit. A first output unit 100A shown in Fig. 3 includes an encoding processing unit 110 and a printing apparatus 112. In the first output unit 100A shown in Fig. 3, the one-packaging medicine information is encoded, and the encoded one-packaging medicine information is printed to a paper medium.

An example of the encoding of the one-packaging medicine information is a one-dimensional encoding or a two-dimensional encoding. Fig. 1 shows a mode in which the one-packaging medicine information is encoded into the two-dimensional code 3 and is printed to the paper medium 4. The printer 33A in Fig. 1 corresponds to the printing apparatus 112 in Fig. 2.

The two-dimensional code 3 in which the one-packaging medicine information printed to the paper medium 4 shown in Fig. 1 by using the printing apparatus 112 shown in Fig. 3 is represented can be read by using the camera included in the mobile terminal apparatus 2 shown in Fig. 1. The camera illustrated by the reference numeral 2B in Fig. 14 and the like is one embodiment of a one-packaging medicine information acquisition unit.

An image of the medicines subjected to the one-packaging processing may be printed by using the printer 33A in Fig. 1. For example, the second imaging data 32B obtained by imaging by using the second camera 32 or the third imaging data 32C obtained by imaging using the third camera 32A may be printed.

The mobile terminal apparatus 2 can acquire the image of the medicines printed by using the printer 33A in Fig. 1 by imaging by using the camera of the mobile terminal apparatus 2.

Fig. 4 is a block diagram showing another configuration example of the first output unit. The first output unit 100B shown in Fig. 4 includes a data conversion processing unit 120 and a data output unit 122. In the first output unit 100B shown in Fig. 4, the one-packaging medicine information is converted into communicable data and is output from the data output unit 122.

The data that is output from the data output unit 122 and in which the one-packaging medicine information is represented can be acquired using a data communication function included in the mobile terminal apparatus 2 shown in Fig. 1.

An example of the data output unit 122 shown in Fig. 3 is a data communication terminal or a data communication apparatus. An output mode of the one-packaging medicine information in the first output unit 100 shown in Fig. 2 is not limited to the mode shown in Figs. 3 and 4 as long as the mode has a format capable of being acquired by the mobile terminal apparatus 2 shown in Fig. 1.

Although an illustration is omitted, another example of the first output unit 100 shown in Fig. 2 is the display unit 34. A case where the display unit 34 is employed as the first output unit 100 is as described above.

### [Description of Configuration Example of Second Output Unit]

Fig. 5 is a block diagram showing a configuration example of the second output unit. The second output unit 102 shown in Fig. 5 includes an imaging data conversion processing unit 114 and an imaging data output unit 116. In the imaging data conversion processing unit 114, the second imaging data 32B or the third imaging data 32C shown in Fig. 2 is converted into a data format corresponding to a storage format of the imaging data in the imaging data storage apparatus 1.

In the imaging data conversion processing unit 114, correlation processing with the one-packaging medicine information is subjected to the second imaging data 32B or the third imaging data 32C shown in Fig. 2.

In the imaging data output unit 116 shown in Fig. 5, imaging data that is the imaging data converted by using the imaging data conversion processing unit 114 and is correlated with the one-packaging medicine information is output. An example of the imaging data output unit 116 is the data communication terminal or the data communication apparatus.

The output mode of the one-packaging medicine information in the second output unit 102 shown in Fig. 2 is not limited to the mode shown in Fig. 5 as long as the data can be stored in the image data storage apparatus 1 shown in Fig. 1.

### [Detailed Description of One-packaging Medicine Audit]

Next, the one-packaging medicine audit will be described in detail.

### <Description of Put-in Medicine Type Correct or Incorrect Determination Processing>

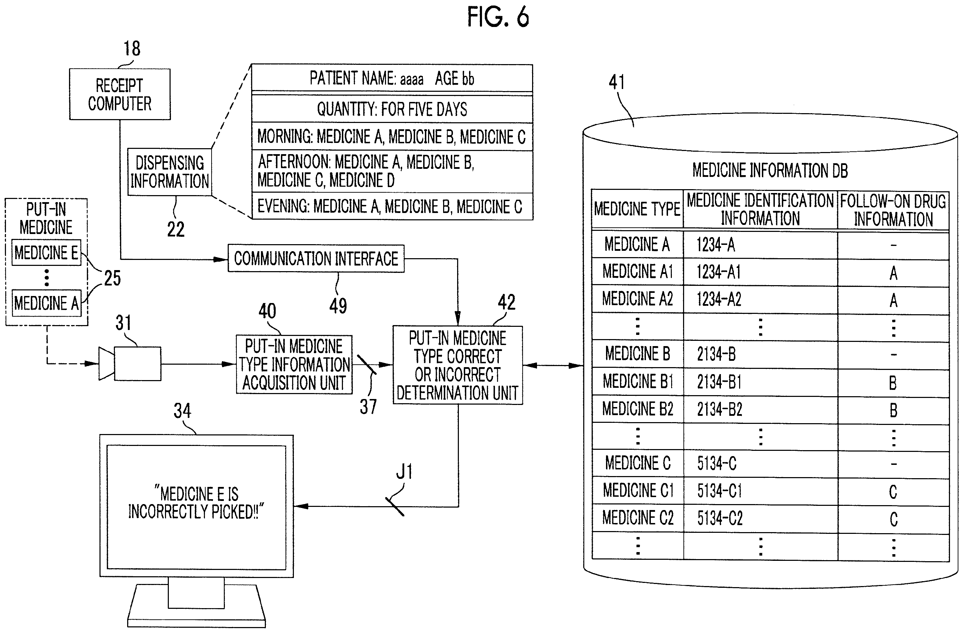

Fig. 6 is an explanatory diagram of correct or incorrect determination processing by using the put-in medicine type correct or incorrect determination unit. In the medicine information database 41 shown in Fig. 6, the medicine type of the medicine 25, the medicine identification information, and follow-on drug information indicating the correspondence relationship between the original drug and the follow-on drug are stored in association with each other in advance. The follow-on drug is sometimes referred to as a generic drug.

A, A1, A2, B, B1, B2, C, C1, and C2 described in a medicine type field of the medicine information database 41 shown in Fig. 6 indicate the medicine types of the medicines 25. The medicines A1 and A2 are the follow-on drugs of a medicine A. The medicines B1 and B2 are the follow-on drugs of a medicine B. The medicines C1 and C2 are the follow-on drugs of a medicine C.

Hereinafter, in a case where it is described as the medicine 25, all medicine types are included. In a case where it is described as the medicines A, B, or the like, the medicine type thereof is indicated.

The medicine type information 37 of the package 36 is stored in a medicine identification information field of the medicine information database 41 shown in Fig. 6. In a case where the medicine 25 is the follow-on drug, the medicine type or the medicine type information 37 of the original drug corresponding to the follow-on drug is stored in a follow-on drug information field. Fig. 6 illustrates an example that the medicine type information 37 is stored in the follow-on drug information field.

With reference to the medicine information database 41, the medicine type of the medicine 25 becomes clear from the medicine type information 37, and further it is possible to distinguish whether the medicine 25 is the original drug or the follow-on drug.

In the put-in medicine type correct or incorrect determination unit 42, the medicine type of the medicine 25 required to be put into the one-packaging processing apparatus 26 is distinguished based on the dispensing information 22. That is, in the put-in medicine type correct or incorrect determination unit 42, the medicine types of the medicines 25 picked in the picking step S2 shown in Fig. 1 and to be put into the one-packaging processing apparatus 26 are distinguished with reference to the medicine information database 41 based on the medicine type information 37. In Fig. 6, the medicine 25 to be put into the one-packaging processing apparatus 26 is described as a put-in medicine.

In the put-in medicine type correct or incorrect determination unit 42, medicine types of the medicines 25 designated by the dispensing information 22 are collated with the medicine types of the medicines 25 to be put into the one-packaging processing apparatus 26 to determine whether the medicine types of the medicines 25 to be put into the one-packaging processing apparatus 26 are correct.

In the put-in medicine type correct or incorrect determination unit 42, the medicines that match the medicine types of the medicines 25 designated by the dispensing information 22 among the medicine types of the medicines 25 to be put into the one-packaging processing apparatus 26 are determined to be correct. On the other hand, the medicines that do not match the designated medicine types of the medicines 25 are determined to be incorrect.

In the put-in medicine type correct or incorrect determination unit 42, in a case where the medicines 25 corresponding to the medicine type information 37 have the same active ingredients as the medicines 25 designated by the dispensing information 22 in the case of the determination, the medicine types are determined to be correct even in a case where both medicine types do not match each other.

An example of the case where both medicines have the same active ingredient is a case where both medicines are the follow-on drugs or a case where both medicines are the same type and effect medicines having the same active ingredient. Although an illustration is omitted, the medicine information database 41 stores in advance information indicating a correspondence relationship between the same type and effect medicines.

The medicine type information 37 which is the information of the medicine type field stored in the medicine information database 41 and the information of the medicine identification information field can be employed as the one-packaging medicine information. In the one-packaging medicine information, in a case where the original drug described on the prescription is replaced with the follow-on drug, the information representing the original drug is replaced with the information representing the follow-on drug.

In the put-in medicine type correct or incorrect determination unit 42, in a case where the medicine types of the medicines 25 to be put into the one-packaging processing apparatus 26 are determined to be incorrect, the determination result J1 including the information on the medicine type is output to the display unit 34. The display unit 34 displays that the medicine types of the medicines 25 to be put into the one-packaging processing apparatus 26 are incorrect and the medicine type thereof.

For example, in a case where the medicine type information 37 on the package 36 of a medicine E not designated by the dispensing information 22 is imaged by using the first camera 31, the determination result J1 that the medicine E is incorrect is output from the put-in medicine type correct or incorrect determination unit 42 to the display unit 34.

The display unit 34 displays warning information indicating that the medicine E is incorrectly picked. A display mode of the warning display may be changed as appropriate.

### <Description of Appearance-based Number Correct or Incorrect Determination Processing>

Fig. 7 is an explanatory diagram of the correct or incorrect determination processing by using the appearance-based number correct or incorrect determination unit. The medicine type of the medicine 25 and the medicine appearance information indicating the appearance of the medicine are stored in association with each other in the medicine appearance database 43 shown in Fig. 7.

The medicine appearance information includes information indicating the length, the shape, the color of the medicine 25. An example of the length of the medicine 25 is the area, the diameter, the radius, the length of the major axis, or the length of the minor axis.

An example of the shape of the medicine 25 is the circle, the triangle, the ellipse, or the capsule shape. An example of the color of the medicine 25 is the RGB value. L1, L2, L3, and L4 indicating respective lengths of the medicine A, the medicine B, the medicine C, and a medicine D shown in Fig. 7, S1, S2, S3, and S4 indicating respective shapes of the above medicines, and C1, C2, C3, and C4 indicating respective colors of the above medicines are known values.

In the appearance-based number information acquisition unit 44, appearance-based number information 53 indicating the appearance-based number which is the number of the medicines 25 to be one-packaged in the one-packaging bags 27 by the appearance is acquired with reference to the medicine appearance database 43 based on the one-packaging bag medicine information 51 input from the one-packaging bag medicine information acquisition unit 50.

For example, in the appearance-based number information acquisition unit 44, in a case where the one-packaging bag medicine information 51 corresponding to a one-packaging bag α is one medicine A, one medicine B, and one medicine C, the appearance-based number information 53 on the one-packaging bag α is decided with reference to the pieces of medicine appearance information corresponding to the medicine A, the medicine B, and the medicine C of the medicine appearance database 43.

In the case, the appearance-based number information 53 on the one-packaging bag α is one medicine having the length of L1, the shape of S1, and the color of C1, one medicine having the length of L2, the shape of S2, and the color of C2, and one medicine having the length of L3, the shape of S3, and the color of C3.

Similarly, in the appearance-based number information acquisition unit 44, the pieces of appearance-based number information 53 corresponding to the one-packaging bag β and the one-packaging bag γ are decided. In the appearance-based number information acquisition unit 44, the pieces of appearance-based number information 53 on the one-packaging bag β and the one-packaging bag γ are output to the appearance-based number correct or incorrect determination unit 46.

In the embodiment, the appearance-based number information 53 is acquired by the appearance-based number information acquisition unit 44 based on the one-packaging bag medicine information 51 acquired from the one-packaging bag medicine information acquisition unit 50. However, the appearance-based number information acquisition unit 44 may have the function of the one-packaging bag medicine information acquisition unit 50.

That is, the one-packaging bag medicine information 51 may be acquired by the appearance-based number information acquisition unit 44 with reference to the medicine information database 41 based on the dispensing information 22 and the medicine type information 37 acquired from the put-in medicine type correct or incorrect determination unit 42 and the like without providing the one-packaging bag medicine information acquisition unit 50. Accordingly, in the appearance-based number information acquisition unit 44, the appearance-based number information 53 can be acquired based on at least the dispensing information 22 and the medicine type information 37. The dispensing information 22 and the medicine type information 37 are shown in Fig. 6.

In the appearance-based counting unit 45 shown in Fig. 7, the second imaging data 32B input from the second camera 32 or the third imaging data 32C input from the third camera 32A not shown in Fig. 7 is analyzed, and the appearance-based number of the medicines 25 to be one-packaged in each one-packaging bag 27 is counted. Fig. 7 shows an example that the second imaging data 32B input from the second camera 32 is analyzed.

That is, in the appearance-based counting unit 45, the number of medicines 25 is counted for each item corresponding to the medicine appearance information such as the length of the medicine, the shape of the medicine, and the color of the medicine. The appearance-based counting unit 45 may be provided integrally with the second camera 32 or the third camera 32A not shown in Fig. 7.

Specifically, in the appearance-based counting unit 45, publicly known edge extraction processing or segmentation processing is subjected to the second imaging data 32B or the third imaging data 32C not shown in Fig. 7. As a result of the edge extraction processing or the segmentation processing, outlines of the medicines 25 of the second imaging data 32B or the third imaging data 32C not shown in Fig. 7 are extracted. The length of the medicine 25 and the shape of the medicine 25 can be distinguished using the outlines of the medicines 25.

In a case where the second imaging data 32B or the third imaging data 32C not shown in Fig. 7 is color image data, the appearance-based counting unit 45 can distinguish the colors of the medicines 25 based on the second imaging data 32B or the third imaging data 32C not shown in Fig. 7.

The method of counting the appearance-based number of the medicines 25 based on the second imaging data 32B or the third imaging data 32C not shown in Fig. 7 is not limited to the example shown in the embodiment, and various publicly known methods may be used.

In the appearance-based counting unit 45, appearance-based number actual measurement information 55 indicating a counting result of the appearance-based number is output to the appearance-based number correct or incorrect determination unit 46. Hereinafter, the appearance-based number actual measurement information 55 is sometimes described as actual measurement information 55.

The actual measurement information 55 is information having the same format as the appearance-based number information 53. The counting result for each one-packaging bag 27 is recorded in the actual measurement information 55.

In the appearance-based number correct or incorrect determination unit 46, the appearance-based number information 53 output from the appearance-based number information acquisition unit 44 is collated with the actual measurement information 55 output from the appearance-based counting unit 45. The determination result J2 is output to the one-packaging correct or incorrect determination unit 48.

The one-packaging bag medicine information 51 and the actual measurement information 55 are the components of the one-packaging medicine information. The appearance-based number information 53 may be the component of the one-packaging medicine information instead of the actual measurement information 55.

### <Description of Mix-up Determination>

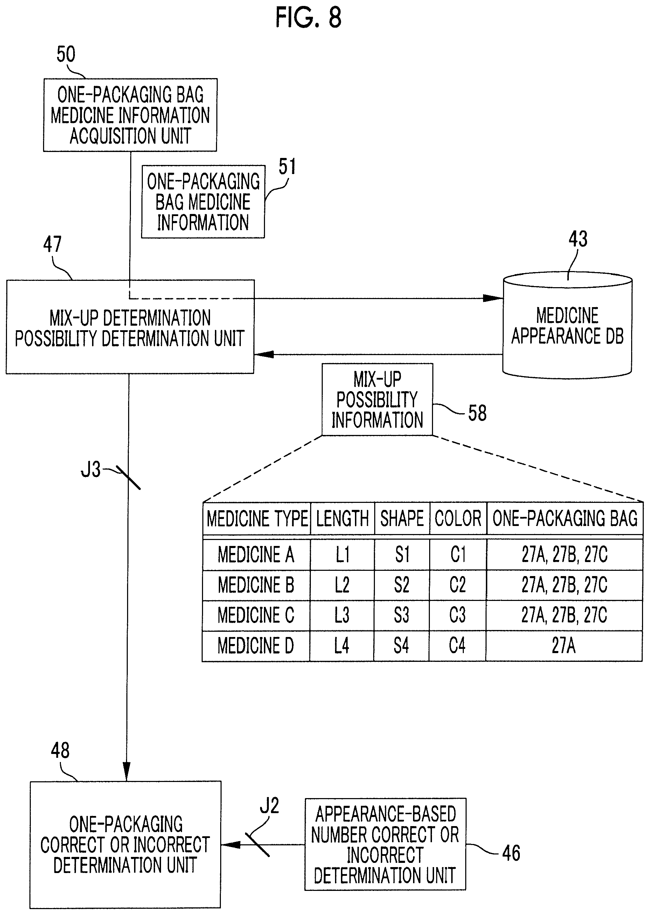

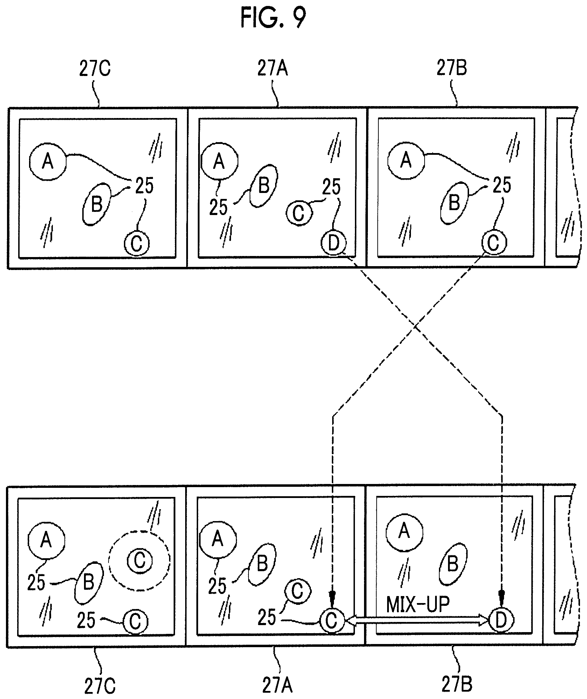

Fig. 8 is an explanatory diagram of determination processing by using a mix-up determination possibility determination unit. Fig. 9 is an explanatory diagram of a mix-up in the medicines.

The mix-up of the medicines 25 means that the different type of medicine 25 is input to individual one-packaging bag 27 instead of the medicine 25 required to be input to individual one-packaging bag 27. The different type of medicine herein excludes the same type and effect medicine such as the follow-on drug.

As shown in Fig. 9, the mix-up between the one-packaging bags 27 in which the medicine D required to be input to the one-packaging bag 27A is input to the one-packaging bag 27B, and the medicine C required to be input to the one-packaging bag 27B is conversely input to the one-packaging bag 27A is an example.

The mix-up does not occur only between the one-packaging bags, and, for example, the mix-up within the same one-packaging bag in which the medicine C is input to the one-packaging bag 27A instead of the medicine D originally required to be input to the one-packaging bag 27A, and the medicine D is not input to any of other one-packaging bags 27 is also an example. Fig. 9 also shows an example that one extra medicine C is input to the one-packaging bag 27C.

The mix-up occurs due to a setting mistake of the medicine 25 on the tray of the one-packaging processing apparatus 26 shown in Fig. 1, an error of the one-packaging processing apparatus 26, or the like.

Whether the determination of the presence or absence of the mix-up is possible means whether the determination of the presence or absence of the mix-up is possible from the appearance of the medicine 25. For example, in a case where the mix-up occurs between the medicine C and the medicine D in the one-packaging bag 27A and the one-packaging bag 27B shown in Fig. 9, it is possible to determine that the appearance-based number of the medicines 25 to be one-packaged in the one-packaging bags 27A and the one-packaging bag 27B in the appearance-based number correct or incorrect determination unit 46 is incorrect in a case where the appearances of the medicine C and the medicine D are different from each other.

On the other hand, in a case where the appearances of the medicine C and the medicine D are the same or are similar, there is a possibility that the appearance-based counting unit 45 cannot distinguish between the medicine C and the medicine D in the counting. In such case, since the mix-up is not reflected in the actual measurement information 55 even though the mix-up occurs between the medicine C and the medicine D, it is impossible to accurately determine whether the appearance-based number of the medicines 25 is correct by the appearance-based number correct or incorrect determination unit 46.

In the case, since it is impossible to determine the presence or absence of the mix-up, there is a possibility of the mix-up of the different types of medicines 25 even in a case where the appearance-based number correct or incorrect determination unit 46 determines that the appearance-based number of the medicines 25 is correct. As described above, whether the determination of the presence or absence of the mix-up is possible is decided by whether two or more types of medicines having the same or similar appearance are included among the medicines 25 to be put into the one-packaging processing apparatus 26.

In the mix-up determination possibility determination unit 47, mix-up possibility information 58 indicating whether the determination of the presence or absence of the mix-up in the medicines 25 is possible is acquired with reference to the medicine appearance database 43 based on the one-packaging bag medicine information 51 input from the one-packaging bag medicine information acquisition unit 50.

Specifically, the medicine appearance information corresponding to the medicine 25 represented by the one-packaging bag medicine information 51 is acquired by the mix-up determination possibility determination unit 47 as the mix-up possibility information 58 with reference to the medicine appearance database 43 based on the one-packaging bag medicine information 51.

Information indicating a one-packaging bag 27 where the medicine 25 is to be put into is stored in a one-packaging bag field in the mix-up possibility information 58.

Next, in the mix-up determination possibility determination unit 47, it is determined whether the determination of the presence or absence of the mix-up in the medicines 25 to be one-packaged in the one-packaging bags 27 is possible based on the mix-up possibility information 58.

Specifically, in the mix-up determination possibility determination unit 47, it is determined whether the appearance of the medicine 25 satisfies the following first condition to third condition. The number of conditions increases or decreases according to the number of parameters of the appearance-based number.

The first condition is that a difference in the shape of the different types of medicines 25 is small. For example, in the mix-up determination possibility determination unit 47, in a case where the shape of the different type of medicine 25 is represented by a difference between a diameter of the circumscribed circle of the medicine and a diameter of the inscribed circle of the medicine, the difference in the shape of the different types of medicines 25 is determined to be small in a case where the difference between the medicines is less than 0.1 mm, or in a case where the shape of the different type of medicine 25 is represented by the maximum distance between the circumscribed circle of the medicine and the outer edge of the medicine, the difference in the shape of the different types of medicines 25 is determined to be small in a case where the difference between the medicines is less than 0.1 mm.

The second condition is that a difference in the length of the different types of medicines 25 is small. For example, in the mix-up determination possibility determination unit 47, in a case where a difference between the medicines in the diameter of the different type of medicine 25 is less than 0.1 mm or in a case where a difference between the medicines in the minimum distance from the center of the different type of medicine 25 to the outer edge thereof is less than 0.05 mm, the difference in the length of the different types of medicines 25 is determined to be small. The center of the medicine 25 is a point at which a sum of distances from the entire periphery of the outer edge is the shortest.

The third condition is that differences in the color and brightness of the different types of medicines 25 are small. For example, in the mix-up determination possibility determination unit 47, in a case where a color difference between the medicines under the standard light source is less than 2.0 or in a case where a difference between the medicines in the all RGB values is less than 10%, the differences in the color and the brightness of the different types of medicines 25 are determined to be small. A publicly known technique set by International Commission on Illumination or the like may be used as a method of calculating the color difference.

In the mix-up determination possibility determination unit 47, in a case where the appearances of the different types of medicines 25 respectively to be put into the different one-packaging bags 27 satisfies the first condition to the third condition, the determination of the presence or absence of the mix-up in the medicines 25 is determined to be impossible based on the mix-up possibility information 58.

In the mix-up determination possibility determination unit 47, in the case where the determination of the presence or absence of the mix-up in the different types of medicines 25 is impossible, the determination result J3 including the medicine types of the different types of medicines 25 is generated.

On the other hand, in the mix-up determination possibility determination unit 47, in a case where the appearances of the different types of medicines 25 respectively to be put into the different one-packaging bags 27 do not satisfy any of the first condition to the third condition, the determination of the presence or absence of the mix-up is determined to be possible, and the determination result J3 is generated.

In the mix-up determination possibility determination unit 47, the same determination is made whether the different types of medicines 25 to be put into the same one-packaging bag 27 satisfy the first condition to the third condition, and the determination result J3 is generated.

In a case where the mix-up determination possibility determination unit 47 determines that the determination of the presence or absence of the mix-up in the different types of medicines 25 is impossible, the medicine types of the different types of medicines 25 are included in the determination result J3.

In the mix-up determination possibility determination unit 47, the determination result J3 is output to the one-packaging correct or incorrect determination unit 48. In the embodiment, the mix-up determination possibility determination unit 47 acquires the mix-up possibility information 58 based on the one-packaging bag medicine information 51 acquired from the one-packaging bag medicine information acquisition unit 50 and determines whether the determination of the presence or absence of the mix-up in the medicines 25 is possible. However, the mix-up determination possibility determination unit 47 may have the function of the one-packaging bag medicine information acquisition unit 50.

That is, the mix-up possibility information 58 may be acquired by the mix-up determination possibility determination unit 47 with reference to the medicine appearance database 43 based on the dispensing information 22 and the medicine type information 37 acquired from the put-in medicine type correct or incorrect determination unit 42 and the like without providing the one-packaging bag medicine information acquisition unit 50.

Accordingly, in the mix-up determination possibility determination unit 47, it is possible to determine whether the determination of the presence or absence of the mix-up in the medicines 25 is possible based on at least the dispensing information 22 and the medicine type information 37.

### [Description of Warning Display]

Fig. 10 is an explanatory diagram of an example of a warning display in a case where the appearance-based number correct or incorrect determination unit determines that it is incorrect. In the previously described appearance-based number correct or incorrect determination, in a case where the appearance-based number is determined to be incorrect, the warning information shown in Fig. 10 is displayed on the display unit 34.

Fig. 11 is an explanatory diagram of an example of the warning display in a case where the mix-up determination possibility determination unit determines that the determination of the presence or absence of the mix-up is impossible. In the previously described mix-up determination, in the case where it is determined that the determination of the presence or absence of the mix-up is impossible, the warning information shown in Fig. 11 is displayed on the display unit 34.

### [Description of Flow of Procedure for One-packaging Medicine Audit]

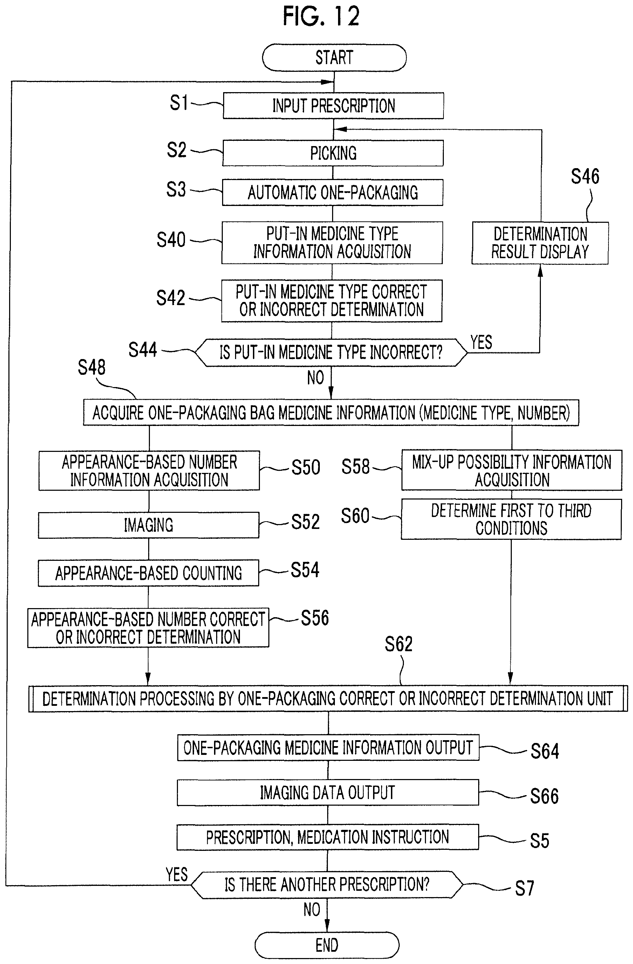

Next, a flow of a procedure for the one-packaging medicine audit will be described. Fig. 12 is a flowchart showing the procedure for the one-packaging medicine audit including a method of outputting the one-packaging medicine information.

In the prescription input step S1 shown in Fig. 12, the dispensing information 22 is input to the receipt computer 18, and the dispensing information 22 is output by using the printer 19 shown in Fig. 1. In the prescription input step S1 shown in Fig. 12, the dispensing information 22 shown in Fig. 1 is output from the receipt computer 18 to the one-packaging medicine audit apparatus 20. Accordingly, it is possible to acquire the dispensing information 22 in the one-packaging medicine audit apparatus 20.

In the picking step S2 shown in Fig. 12, the medicine 25 corresponding to the dispensing information 22 output from the printer 19 shown in Fig. 1 is picked from the medicine shelf 24. After the picking step S2 shown in Fig. 12, the procedure proceeds to the automatic one-packaging step S3. In parallel with the automatic one-packaging step S3, the one-packaging dispensing audit step S4 shown in Fig. 1 is executed.

In the automatic one-packaging step S3 shown in Fig. 12, the one-packaging processing is performed by using the one-packaging processing apparatus 26 shown in Fig. 1. In the one-packaging dispensing audit step S4 shown in Fig. 1, the following procedure is employed.

In a put-in medicine type information acquisition step S40 shown in Fig. 12, after the picking step S2, the package 36 of the picked medicine 25 is set in front of the first camera 31 shown in Fig. 1. The package 36 is shown in Fig. 1.

The medicine type information 37 of the medicine 25 is imaged by using the first camera 31. The first imaging data 31A which is the imaging data of the medicine type information 37 is output from the first camera 31 to the put-in medicine type information acquisition unit 40. The medicine type information 37 is extracted by using the put-in medicine type information acquisition unit 40 shown in Fig. 2 from the first imaging data 31A.

In a put-in medicine type correct or incorrect determination step S42 shown in Fig. 12, the medicine type information 37 output from the put-in medicine type information acquisition unit 40 is acquired by using the put-in medicine type correct or incorrect determination unit 42 shown in Fig. 2. In the put-in medicine type correct or incorrect determination step S42 shown in Fig. 12, it is determined whether the medicine type of the medicine 25 put into the one-packaging processing apparatus 26 shown in Fig. 1 is correct with reference to the medicine information database 41 based on the medicine type information 37 and the dispensing information 22.

In the put-in medicine type correct or incorrect determination step S42 shown in Fig. 12, in a case where the medicine types of the medicines 25 to be put into the one-packaging processing apparatus 26 shown in Fig. 1 are the original drugs, the follow-on drugs, or the like that are the same or have the same active ingredients as the medicine types of the medicines 25 designated by the dispensing information 22, the medicine types of the medicines 25 to be put into the one-packaging processing apparatus 26 are determined to be correct. In the case, a determination in step S44 shown in Fig. 12 is a NO determination.

On the other hand, in the put-in medicine type correct or incorrect determination step S42 shown in Fig. 12, in a case where the medicine types of the medicines 25 to be put into the one-packaging processing apparatus 26 shown in Fig. 1 do not match the medicine types of the medicines 25 designated by the dispensing information 22 or are not the original drugs or the follow-on drugs thereof, the medicine types of the medicines 25 to be put into the one-packaging processing apparatus 26 are determined to be incorrect. In the case, the determination in step S44 shown in Fig. 12 is a YES determination.

In the case where the determination in step S44 is the YES determination, the procedure proceeds to a determination result display step S46. In the determination result display step S46 shown in Fig. 12, the display unit 34 shown in Fig. 1 displays that the medicine types of the medicines 25 to be put into the one-packaging processing apparatus 26 are incorrect and the medicine type thereof. Accordingly, the incorrection in the picking step S2 shown in Fig. 12 is warned, and it is possible to urge a redoing of the picking step S2.

Hereinafter, in a case where the medicine types of the medicines 25 to be put into the one-packaging processing apparatus 26 shown in Fig. 1 are correct, pieces of processing from the picking step S2 to the determination result display step S46 shown in Fig. 12 are repeated before the determination is made by the put-in medicine type correct or incorrect determination unit 42 shown in Fig. 2.

Even in a case where all medicine types of the medicines 25 are determined to be correct by the put-in medicine type correct or incorrect determination unit 42 shown in Fig. 2, the display unit 34 may display the fact. After the confirmation of the medicine type, the picked medicine 25 is removed from the package 36 shown in Fig. 1 and set on the tray of the one-packaging processing apparatus 26 for each package.

In the case where the determination in step S44 shown in Fig. 12 is the NO determination, the procedure proceeds to a one-packaging bag medicine information acquisition step S48. In the one-packaging bag medicine information acquisition step S48, the one-packaging bag medicine information 51 on the one package of medicines 25 is acquired by the one-packaging bag medicine information acquisition unit 50 with reference to the medicine information database 41 based on the dispensing information 22 and the medicine type information 37 of the medicine 25.

In the one-packaging bag medicine information acquisition step S48, the one-packaging bag medicine information 51 is output by the one-packaging bag medicine information acquisition unit 50 shown in Fig. 2 to the appearance-based number information acquisition unit 44 and the mix-up determination possibility determination unit 47.

An appearance individual determination step includes an appearance-based number information acquisition step S50, an imaging step S52, an appearance-based counting step S54, and an appearance-based number correct or incorrect determination step S56 shown in Fig. 12.

In the appearance-based number information acquisition step S50, the appearance-based number information 53 is acquired by the appearance-based number information acquisition unit 44 shown in Fig. 7 with reference to the medicine appearance database 43 based on the one-packaging bag medicine information 51 input from the one-packaging bag medicine information acquisition unit 50. In the appearance-based number information acquisition step S50 shown in Fig. 12, the appearance-based number information 53 shown in Fig. 7 is output to the appearance-based number correct or incorrect determination unit 46.

In the imaging step S52 shown in Fig. 12, the medicines 25 to be packaged in the one-packaging bag 27 set on the tray of the one-packaging processing apparatus 26 are imaged by using the second camera 32 shown in Fig. 1. The second imaging data 32B which is the image data of the medicines 25 to be packaged in the one-packaging bag 27 is output to the appearance-based counting unit 45.

In the appearance-based counting step S54 shown in Fig. 12, the second imaging data 32B is analyzed and the appearance-based number of the medicines 25 to be packaged in the one-packaging bag 27 is counted by the appearance-based counting unit 45 shown in Fig. 7. The actual measurement information 55 indicating the counting result is output to the appearance-based number correct or incorrect determination unit 46.

In the appearance-based number correct or incorrect determination step S56 shown in Fig. 12, the appearance-based number information 53 input from the appearance-based number information acquisition unit 44 is collated with the actual measurement information 55 input from the appearance-based counting unit 45 to determine whether the appearance-based number of the medicines 25 to be one-packaged in the one-packaging bags 27 is correct by the appearance-based number correct or incorrect determination unit 46 shown in Fig. 7.

In the appearance-based number correct or incorrect determination unit 46, in a case where the appearance-based number of the medicines 25 to be one-packaged in the one-packaging bags 27 is correct, the determination result J2 indicating the fact is output to the one-packaging correct or incorrect determination unit 48. On the other hand, in a case where the appearance-based number of the medicines 25 to be one-packaged in the one-packaging bags 27 is incorrect, the determination result J2 indicating the fact and the one-packaging bag 27 is output to the one-packaging correct or incorrect determination unit 48.

A mix-up possibility presence or absence determination propriety determination step is configured including a mix-up possibility information acquisition step S58 and a condition-specific determination step S60 shown in Fig. 12.

In the mix-up possibility information acquisition step S58 shown in Fig. 12, the mix-up possibility information 58 is acquired with reference to the medicine appearance database 43 based on the one-packaging bag medicine information 51 input from the one-packaging bag medicine information acquisition unit 50 shown in Fig. 8. After the mix-up possibility information acquisition step S58 shown in Fig. 12, the procedure proceeds to the condition-specific determination step S60.

In the condition-specific determination step S60, it is determined by the mix-up determination possibility determination unit 47 shown in Fig. 8 whether the appearances of the different types of medicines 25 to be put into the different one-packaging bags 27 or the same one-packaging bag 27 satisfy the first condition to the third condition previously described based on the mix-up possibility information 58.

In the condition-specific determination step S60 shown in Fig. 12, in a case where the determination of the presence or absence of the mix-up in the different types of medicines 25 is possible, the determination result J3 indicating the fact is generated. The determination result J3 is shown in Fig. 8. In a case where the determination of the presence or absence of the mix-up in the different types of medicines 25 is impossible, the determination result J3 indicating the medicine type of the different type of medicine 25 is generated.

In the condition-specific determination step S60 shown in Fig. 12, the determination result J3 is output to the one-packaging correct or incorrect determination unit 48 shown in Fig. 8. After the condition-specific determination step S60 shown in Fig. 12, the procedure proceeds to the one-packaging correct or incorrect determination step S62.

In the one-packaging correct or incorrect determination step S62, the determination result J2 of the appearance-based number correct or incorrect determination step S56 and the determination result J3 of the condition-specific determination step S60 shown in Fig. 12 are acquired by the one-packaging correct or incorrect determination unit 48 shown in Fig. 8. The determination result J2 is shown in Fig. 8.

It is determined whether the medicines 25 one-packaged in the one-packaging bag 27 are correct based on the determination result J2 and the determination result J3.

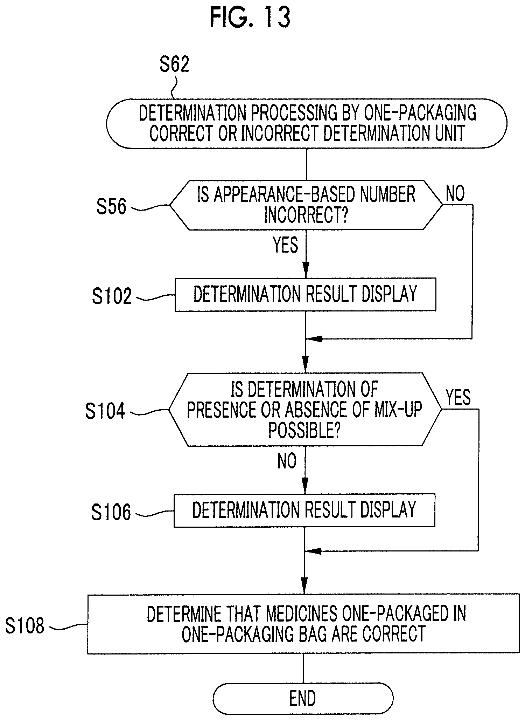

Fig. 13 is a flowchart showing a procedure for determination processing in the one-packaging correct or incorrect determination step. The one-packaging correct or incorrect determination step S62 shown in Figs. 12 and 13 is configured including the appearance-based number correct or incorrect determination step S56, an appearance-based number correct or incorrect determination result display step S102, a mix-up possibility presence or absence determination propriety determination step S104, a mix-up presence or absence determination propriety determination result display step S106, and a one-packaging medicine determination step S108.

In a case of the YES determination in the appearance-based number correct or incorrect determination step S56 shown in Fig. 13, the procedure proceeds to the appearance-based number correct or incorrect determination result display step S102. In the appearance-based number correct or incorrect determination result display step S102, the determination result J2 shown in Fig. 8 is displayed on the display unit 34 shown in Fig. 2.

In a case of the NO determination in the appearance-based number correct or incorrect determination step S56 shown in Fig. 13, the procedure proceeds to the mix-up possibility presence or absence determination propriety determination step S104. In the case of the NO determination in the mix-up possibility presence or absence determination propriety determination step S104, the procedure proceeds to the mix-up presence or absence determination propriety determination result display step S106.

In the mix-up presence or absence determination propriety determination result display step S106, the determination result J3 shown in Fig. 8 is displayed on the display unit 34 shown in Fig. 2. After the mix-up presence or absence determination propriety determination result display step S106 shown in Fig. 13, the procedure proceeds to the one-packaging medicine determination step S108.

In a case of the YES determination in the mix-up possibility presence or absence determination propriety determination step S104, the procedure proceeds to the one-packaging medicine determination step S108. The processing of the condition-specific determination step S60 shown in Fig. 12 may be employed as the mix-up possibility presence or absence determination propriety determination step S1040 shown in Fig. 13.

In the one-packaging medicine determination step S108, in a case where the appearance-based number of the medicines 25 to be put into the one-packaging bag 27 is correct and in a case where the determination of the presence or absence of the mix-up in the medicines 25 is possible, the medicines 25 one-packaged in the one-packaging bag 27 are determined to be correct by the one-packaging correct or incorrect determination unit 48 shown in Fig. 8.

In the case, the display unit 34 shown in Fig. 2 also displays information indicating that the medicines 25 one-packaged in the one-packaging bag 27 are correct.

Returning to Fig. 12, in the one-packaging correct or incorrect determination step S62, in the case where the appearance-based number of the medicines 25 to be put into the one-packaging bag 27 is determined to be correct and in the case where the determination of the presence or absence of the mix-up in the medicines 25 is determined to be possible, the procedure proceeds to a one-packaging medicine information output step S64.

In the one-packaging medicine information output step S64, the one-packaging medicine information that is the one-packaging medicine information relating to the medicines 25 one-packaged in the one-packaging bag 27 and is acquired by the one-packaging medicine audit apparatus 20 shown in Fig. 1 is output from the first output unit 100 shown in Fig. 2.

That is, in the one-packaging medicine information output step S64, the one-packaging medicine information of which an audit determination is passed is output. After the one-packaging medicine information output step S64, the procedure proceeds to the imaging data output step S66.

In an imaging data output step S66, at least any one of the second imaging data 32B in which the medicines 25 one-packaged in the one-packaging processing apparatus 26 shown in Fig. 1 are imaged by using the second camera 32 or the third imaging data 32C in which the medicines 25 one-packaged in the one-packaging processing apparatus 26 shown in Fig. 1 are imaged by using the third camera 32A is output to the imaging data storage apparatus 1 through the second output unit 102.

The one-packaging medicine information output from the first output unit 100 can be acquired by using the mobile terminal apparatus 2. At least any one of the second imaging data 32B or the third imaging data 32C output to the imaging data storage apparatus 1 through the second output unit 102 can be used using the medicine notebook application installed in the mobile terminal apparatus 2.

The one-packaging medicine audit by the one-packaging medicine audit apparatus 20 shown in Fig. 1 is completed through the above-described steps. The one-packaging medicine information output step S64 and the imaging data output step S66 shown in Fig. 13 may be executed separately from the one-packaging medicine audit.

After the one-packaging medicine audit, the prescription step S5 is executed. Hereinafter, in an accepting step S7, the steps S1 to S5 are executed every time a new prescription is accepted.

### [Action Effect]

With the one-packaging medicine audit apparatus, the one-packaging medicine audit system, and the method of outputting the one-packaging medicine information configured as described above, since the information relating to the one-packaging medicines acquired by the one-packaging medicine audit apparatus is output as readable information by using the mobile terminal apparatus, even in the case where the two-dimensional code or the like is not printed on the prescription or the medicine bag, it is possible to acquire the one-packaging medicine information by using the mobile terminal apparatus.

Since the one-packaging medicine information acquired by using the one-packaging medicine audit apparatus is output, for example, even in the case where the original drug is replaced with the follow-on drug, is replaced with the same type and effect medicine, or the like, it is possible to acquire the one-packaging medicine information in which the information on the actually prescribed medicine is reflected.

Since the encoded one-packaging medicine information is output, it is possible to easily read the encoded information by using the mobile terminal apparatus.

Since the one-packaging medicine information of which the audit determination is passed is output, it is possible to acquire the one-packaging medicine information of which the audit determination is passed.

Since the imaging data of the medicines actually subjected to the one-packaging processing is output in correlation with the one-packaging medicine information and is stored in an imaging data storage apparatus, the medicine notebook application installed in the mobile terminal apparatus can use the imaging data stored in the imaging data storage apparatus with the one-packaging medicine information as the key information.

The imaging data of the medicines actually subjected to the one-packaging processing may be the imaging data before the medicines are packaged in the one-packaging bag or after the medicines are packaged in the one-packaging bag.

### [Description of Function of Medicine Notebook Application]

Next, the function of the medicine notebook application to be installed in the mobile terminal apparatus will be described using Figs. 14 to 22. A mobile phone, a portable plate computer, or the like can be employed as the mobile terminal apparatus.

### <Description of Problem>

The browsing system described in JP2007-007286A accesses the browsing database by using the mobile terminal apparatus and can browse the information relating to the medicine in the browsing database. However, the browsing system cannot prevent dose forgetting of the medicines, the mix-up of the medicines, or replenishment forgetting of the medicines. The browsing system is not an easily usable application.

It is desired that the digitized medicine notebook application is the easily usable application for relatively elderly people. In the medicine notebook application to be described below, it is possible to prevent the dose forgetting of the medicines, the mix-up of the medicines, or the replenishment forgetting of the medicines using a visual graphical user interface displaying the imaging data of the medicines one-packaged that is imaged in the case of the one-packaging medicine audit.

It can be said that the medicine notebook application to be described below is an easily usable application using an operation of the mobile terminal apparatus.

### <Description of Mobile Terminal Apparatus>

Although a detailed illustration of the configuration of the mobile terminal apparatus is omitted, the mobile terminal apparatus comprises a reading unit, a data communication unit, a display unit, and a program execution unit. A reading apparatus corresponding to an output format of the one-packaging medicine information such as the camera or a code reader can be employed as the reading unit. In Figs. 14 to 22, the camera is illustrated by the reference numeral 2B.

The data communication unit is configured of a data input unit corresponding to a communication format of the one-packaging medicine information and a communication format of the imaging data such as a data input terminal or a data input port, a storage unit that stores the input data, and a data conversion unit that converts the data. In Figs. 14 to 22, the data input terminal is illustrated by a reference numeral 2E.

The display unit is configured of the touch panel 2A that displays a screen and a display driver not shown. The data communication unit is one embodiment of a mobile terminal imaging data acquisition unit.

The program execution unit executes the medicine notebook application installed in the mobile terminal apparatus. The mobile terminal apparatus may comprise a program storage unit that stores the medicine notebook application. The program execution unit is one embodiment of an application execution unit. The program storage unit is one embodiment of an application storage unit.

### <Description of Basic Function of Medicine Notebook Application>

Figs. 14 to 16 are explanatory diagrams showing examples of display screens in a case where a basic function of the medicine notebook application is executed. In the mobile terminal apparatus 2 shown in Fig. 14, the touch panel 2A displays a basic function screen 200 in the case where the medicine notebook application is executed.

A reference numeral 2C shown in Fig. 14 is a speaker. A reference numeral 2D shown in Fig. 14 is a main body frame.

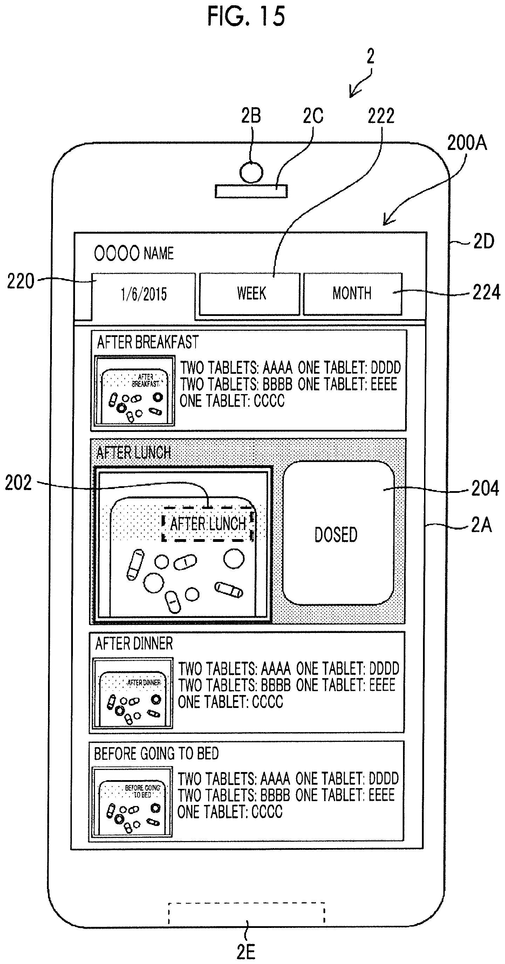

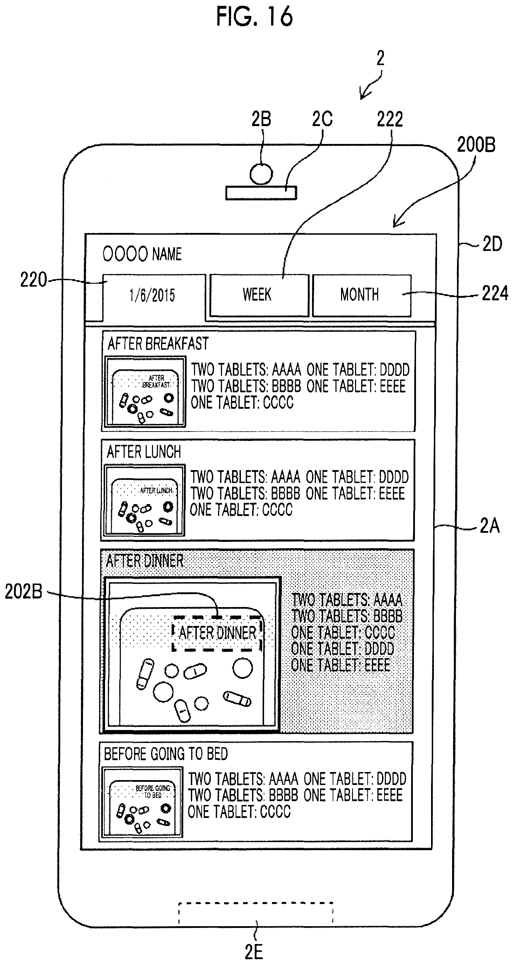

Information on the medicines required to be dosed in a day is displayed in the basic function screen 200 shown in Fig. 14, a basic function screen 200A shown in Fig. 15, and a basic function screen 200B shown in Fig. 16. The basic function screens display the types of medicines for each dose timing of medicines, the number of medicines to be dosed for each type of medicine, and the image of medicines for each one-packaging bag 27. That is, the basic function screens display information for each one-packaging bag subjected to the one-packaging processing.

Each of the types of medicines for each dose timing of medicines, the number of medicines to be dosed for each type of medicine, and the image of the medicines one-packaged displayed on the basic function screen 200 shown in Fig. 14, the basic function screen 200A shown in Fig. 15, and the basic function screen 200B shown in Fig. 16 is a component of the dose information on the medicines subjected to the one-packaging processing.

In Fig. 14, the medicines to be dosed after lunch are enlarged and displayed as compared with the medicines to be dosed after breakfast, after dinner, and after going to bed at the timing after lunch. A highlight display 202 indicating the medicines required to be dosed after lunch is attached by overlapping with the image of the medicines one-packaged. The highlight display 202 is information not attached to the one-packaging bag.

The timing after lunch is automatically recognized using a clock function of the mobile terminal apparatus 2. The timing after lunch may be set in advance.

The image of the medicines dosed after breakfast shown in Fig. 14 is one embodiment of an image represented by the imaging data of the medicines dosed in the past. The images of the medicines to be dosed after dinner and after going to bed are one embodiment of an image represented by the imaging data of the medicines required to be dosed in the future. The image of the medicines to be dosed after lunch is one embodiment of an image represented by the imaging data of the medicines required to be dosed next time.

That is, important information in a case where the medicines are dosed is automatically recognized by the medicine notebook application, and the highlight display 202 representing the important information in the case where the medicines are dosed is added on the image of the medicines one-packaged.

Fig. 15 illustrates the basic function screen 200A displaying that the medicines required to be dosed after lunch are dosed. In a case where an operation such as a swipe operation is performed on the display of the medicines required to be dosed after lunch, dose completion information 204 representing that the medicines required to be dosed after lunch shown in Fig. 15 are dosed is displayed.

In Fig. 16, the medicines to be dosed after dinner are enlarged and displayed as compared with the medicines to be dosed after breakfast, after lunch, and after going to bed at the timing after dinner. The highlight display 202B is attached.

As described above, the medicine notebook application can automatically change the display screen displayed on the touch panel 2A of the mobile terminal apparatus 2 as time elapses.

In the embodiment, the highlight display representing the important information in the case where the medicines are dosed is added on the image of the medicines one-packaged using the function of the medicine notebook application installed in the mobile terminal apparatus 2. However, data corresponding to the highlight display may be attached to the one-packaging medicine information to be output from the one-packaging medicine audit apparatus 20 shown in Fig. 1 as the highlight display.

In Figs. 14 to 16, the embodiment displaying the medicines required to be dosed in units of a day is exemplified. However, the medicines required to be dosed may be displayed in units of a week or in units of a month. Figs. 14 to 16 illustrate a first tab 220, a second tab 222, and a third tab 224 operated in a case where the units displaying the medicines required to be dosed are switched.

In Figs. 14 to 16, the embodiments displaying the medicines currently required to be dosed, the medicines dosed in the past, and the medicines required to be dosed in the future are exemplified. However, at least any one of the medicines dosed in the past or the medicines required to be dosed in the future may be displayed.

### <Description of Urging Function of Medicine Notebook Application>

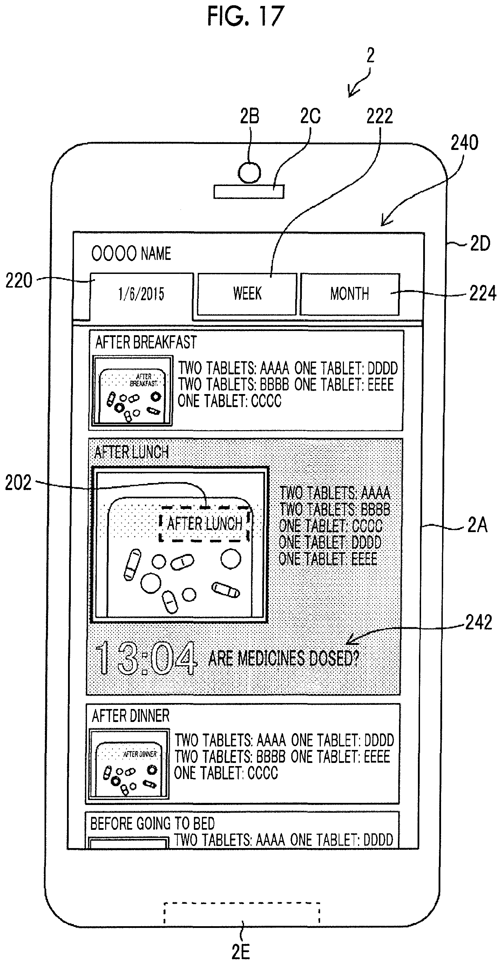

Figs. 17 to 19 are explanatory diagrams showing examples of display screens in a case where an urging function of the medicine notebook application is executed. In the mobile terminal apparatus 2 shown in Fig. 17, the touch panel 2A displays an urging function screen 240 in the case where the medicine notebook application is executed.

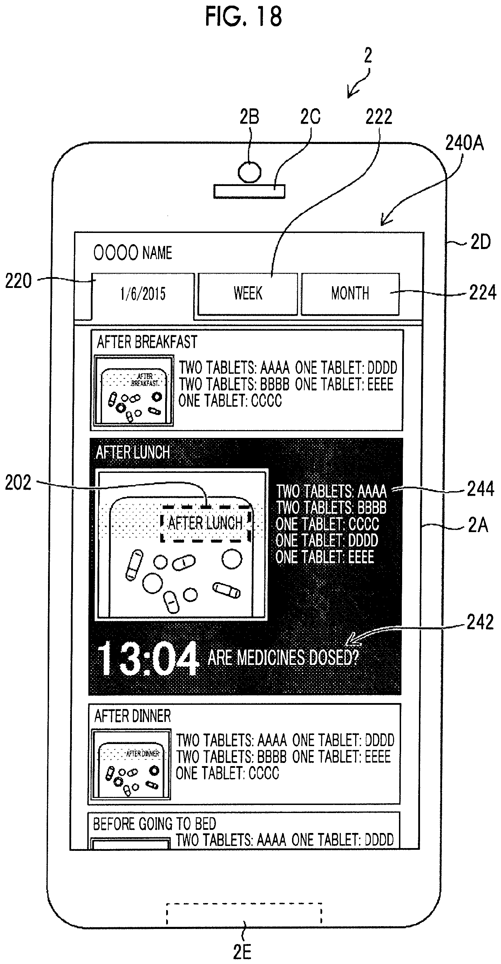

In the urging function screen 240 shown in Fig. 17, an urging display 242 is added to the basic function screen 200 shown in Fig. 14. In the urging function screen 240A shown in Fig. 18, a color of a background 244 of the urging display 242 on the urging function screen 240 shown in Fig. 17 is changed.

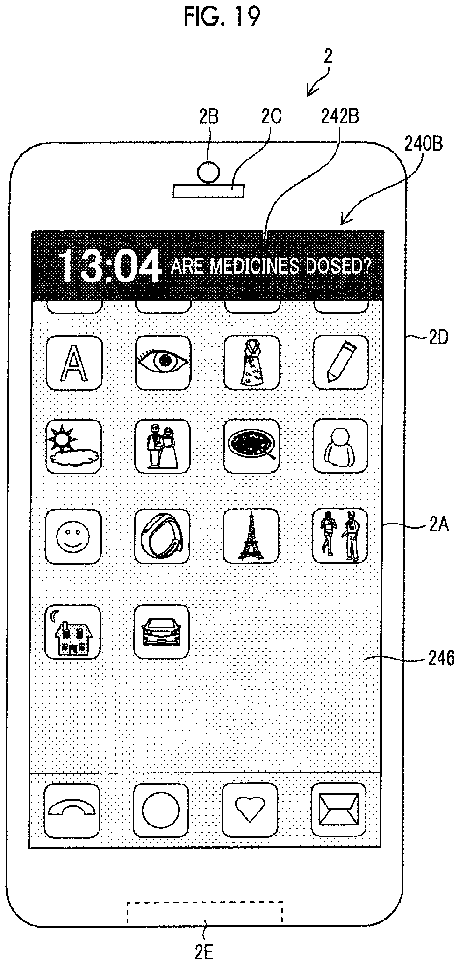

In the urging function screen 240B shown in Fig. 19, the urging display 242B is displayed on the upper portion of a standby screen 246. In the urging function screen 240B shown in Fig. 19, in a case where the urging display 242B is tapped, the urging function screen 240B may be changed to the basic function screen 200 shown in Fig. 14.

A timing where the urging function is executed is set in advance. Timings at which a plurality of urging functions are executed may be set or a plurality of times of urgings may be executed for one urging timing setting.

In the case where the plurality times of urgings are executed, an urging level may be raised as time elapses. For example, the urging function screen 240 shown in Fig. 17 may be displayed in a case of a first urging, and the urging function screen 240A shown in Fig. 18 may be displayed in a case of a second urging. The processing of raising the urging level as time elapses is one embodiment of the processing of urging the dose according to an elapsed period from the dose timing.

### <Medicine Shortage Notification Function>

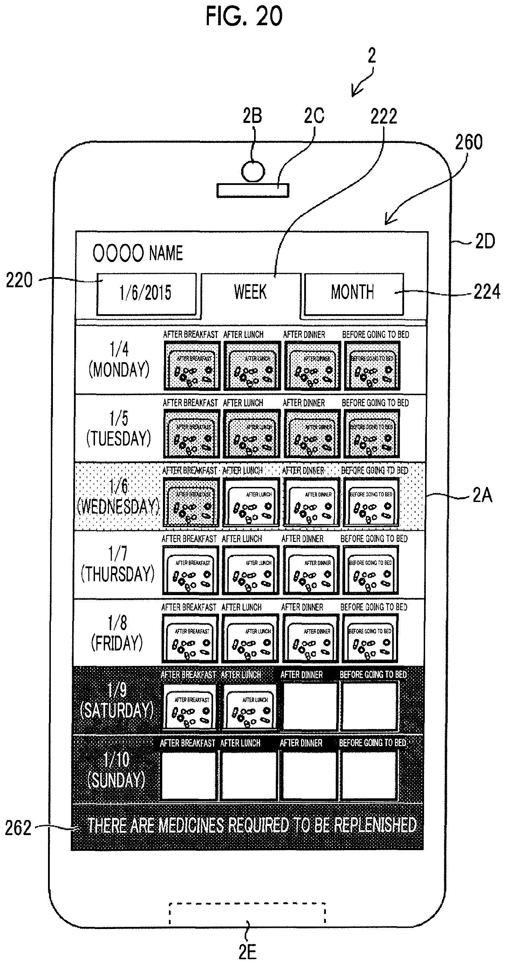

Figs. 20 to 22 are explanatory diagrams showing examples of display screens in a case where a medicine shortage notification of the medicine notebook application is executed. A medicine shortage notification display screen 260 shown in Fig. 20 displays the medicines required to be dosed in units of a week. A medicine shortage notification 262 is also displayed. Furthermore, it is represented that there is a shortage of the medicines to be dosed after dinner on January 9th.

On a medicine shortage notification display screen 260 shown in Fig. 20, images at dose timings where the medicines required to be dosed are insufficient are not displayed. Colors of frames at the dose timings where the images are not displayed are changed to red to be highlighted. On the other hand, images at dose timings already dosed from after breakfast on January 4th to after breakfast on January 9th are changed to inconspicuous displays such as shaded expressions.

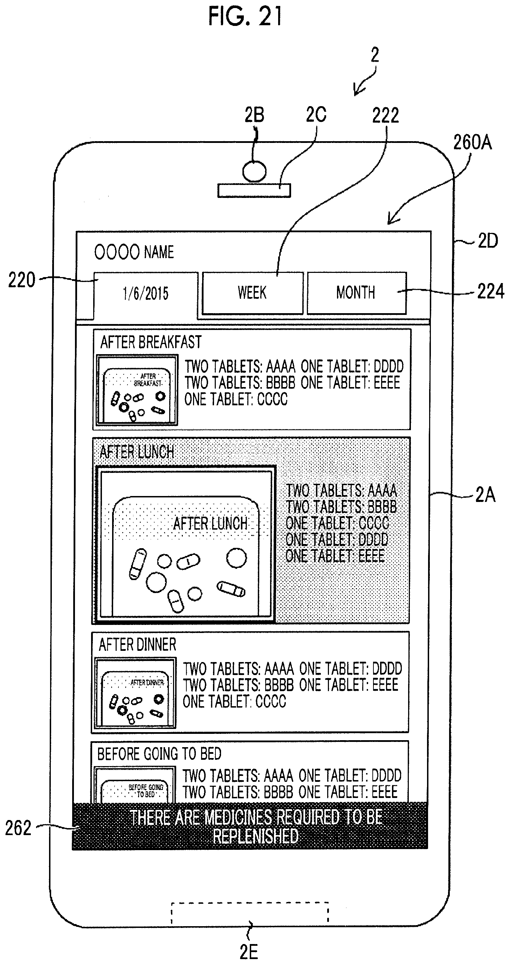

Fig. 21 is the explanatory diagram of another example of the medicine shortage notification display screen. A medicine shortage notification display screen 260A shown in Fig. 21 can be displayed after a bar representing the day of the dose on the medicine shortage notification display screen 260 shown in Fig. 20 is tapped.

In the medicine shortage notification display screen 260A shown in Fig. 21, the medicine shortage notification 262 is added to the basic function screen 200 shown in Fig. 14. Means for notifying the medicine shortage notification 262 in the mobile terminal apparatus 2 is one embodiment of a replenishment medicine specification unit that specifies the type of a medicine required to be replenished using dose history information representing actually dosed medicines.

The means for notifying the medicine shortage notification 262 in the mobile terminal apparatus 2 includes the program execution unit that executes the medicine notebook application, a memory referenced by the medicine notebook application, and a timer.

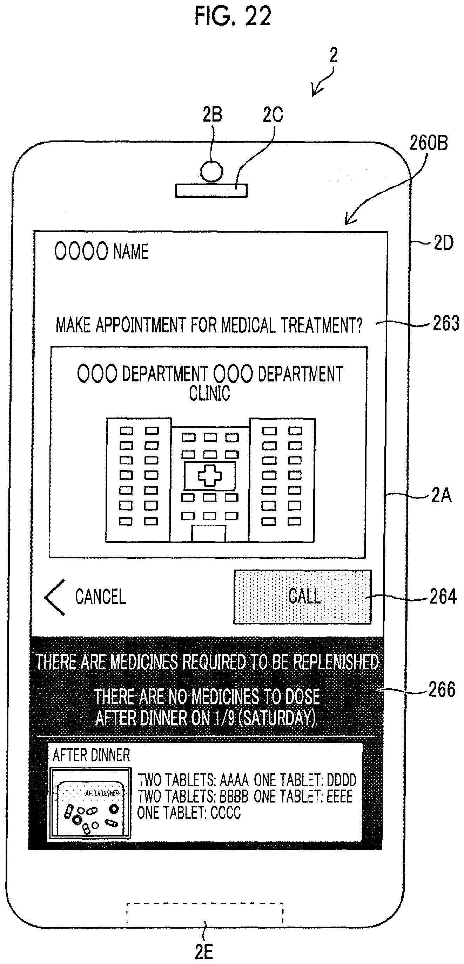

Fig. 22 is the explanatory diagram of still another example of the medicine shortage notification display screen. A medicine shortage notification display screen 260B shown in Fig. 22 can be displayed after the medicine shortage notification 262 of the medicine shortage notification display screen 260A shown in Fig. 21 is tapped.

A button 264 to call a family hospital and an inventory display 266 showing an inventory situation of the medicines in the family hospital are displayed on the medicine shortage notification display screen 260B shown in Fig. 22. A family hospital display 263 showing a name of the family hospital and an image of the appearance of the family hospital is displayed on the medicine shortage notification display screen 260A shown in Fig. 22.

In a case where the button 264 on the medicine shortage notification display screen 260B shown in Fig. 22 is tapped, it is possible to call the family hospital using a call function of the mobile terminal apparatus 2.

The functions of the medicine notebook application described using Figs. 14 to 22 can be added, changed, or deleted as appropriate.

### [Description of Action Effect]

With the medicine notebook application configured as described above, it is possible to prevent the dose forgetting of the medicines, the mix-up of the medicines, or the replenishment forgetting of the medicines using the visual graphical user interface displaying the imaging data of the medicines one-packaged that is imaged in the case of the one-packaging medicine audit.

Since the operation of the mobile terminal apparatus can be used, it is possible to easily use the medicine notebook application.

### [Program Causing Computer to Function as Medicine Inspection Apparatus]

It is possible to store a program for causing a computer to realize the one-packaging medicine information output function of the one-packaging medicine audit apparatus described in the above embodiment in a non-transitory information storage medium as a tangible object which is a CD-ROM, a magnetic disk, or another computer-readable medium and to provide the program through the information storage medium.

For example, it is possible to configure a one-packaging medicine information output program for causing the computer to function as the first output means for outputting the one-packaging medicine information that is related to the medicines 25 actually subjected to the one-packaging processing by using the one-packaging processing apparatus 26 shown in Fig. 1 and is acquired by using the one-packaging medicine audit apparatus 20 as information having a format capable of being acquired by the mobile terminal apparatus 2.

It is also possible to provide a program signal as a download service using a communication network such as the Internet instead of the embodiment in which the program is stored and provided in such information storage medium.

It is also possible to realize a part or all of the one-packaging medicine audit apparatus described in the above embodiment by an application server or cloud computing and to perform a service in which a processing function is provided through a network. The CD-ROM is an abbreviation of compact disc read-only memory.

### Explanation of References

1: imaging data storage apparatus
2: mobile terminal apparatus
2B: camera
2C: speaker
2D: main body frame
2E: data input terminal
3: two-dimensional code
4: paper medium
10: one-packaging medicine information audit system
18: receipt computer
20: one-packaging medicine audit apparatus
19, 33A: printer
22: dispensing information
24: medicine shelf
25, A, A1, A2, B, B1, B2, C, C1, C2, E: medicine
26: one-packaging processing apparatus
27, 27A, 27B, 27C, α, β, γ: one-packaging bag
31: first camera
31A: imaging data
32: second camera
32A: third camera
32B: second imaging data
32C: third imaging data
33: main body portion
34: display unit
36: package
37: medicine type information
40: put-in medicine type information acquisition unit
41: medicine information database
42: put-in medicine type correct or incorrect determination unit
43: medicine appearance database
44: appearance-based number information acquisition unit
45: appearance-based counting unit
46: appearance-based number correct or incorrect determination unit
47: mix-up determination possibility determination unit
48: one-packaging correct or incorrect determination unit
49: communication interface
50: one-packaging bag medicine information acquisition unit
51: one-packaging bag medicine information
53: appearance-based number information
55: actual measurement information
58: mix-up possibility information
100, 100A, 100B: first output unit
102: second output unit
104: image storage unit
110: encoding processing unit
112: printing apparatus
114: imaging data conversion processing unit
116: imaging data output unit
120: data processing unit
122: data output unit
200, 200A, 200B: basic function screen
202, 202B: highlight display
204: dose completion information
220: first tab
222: second tab
224: third tab
240, 240A, 240B: urging function screen
242, 242B: urging display
244: background
246: standby screen
260A, 260B: notification display screen
262: medicine shortage notification
263: family hospital display
264: button
266: inventory display
J1, J2, J3: determination result
J4: one-packaging medicine information
J5: imaging data
S1 to S5, S40 to S108: each step of one-packaging medicine audit method

## Claims

1. A one-packaging medicine audit system comprising a one-packaging medicine audit apparatus, a mobile terminal apparatus, and an imaging data storage apparatus that stores imaging data to be output from the one-packaging medicine audit apparatus,
wherein the one-packaging medicine audit apparatus comprises:
a first output unit that outputs identification information on the medicines actually one-packaged by a one-packaging processing apparatus that performs one-packaging processing of the medicines based on prescription information, as one-packaging medicine information having a format capable of being acquired by a mobile terminal apparatus;
an imaging data acquisition unit that acquires imaging data of medicines actually to be subjected to the one-packaging processing by using the one-packaging processing apparatus; and
a second output unit that outputs the imaging data acquired by using the imaging data acquisition unit and the identification information on the medicines actually subjected to the one-packaging processing by using the one-packaging processing apparatus in correlation with each other,
wherein the mobile terminal apparatus comprises:
a one-packaging medicine information acquisition unit that acquires the one-packaging medicine information output from the one-packaging medicine audit apparatus;
a mobile terminal imaging data acquisition unit that acquires the imaging data correlated with the one-packaging medicine information from the imaging data storage apparatus, by using the one-packaging medicine information acquired by using the one-packaging medicine information acquisition unit; and
a display unit that displays an image for each one-packaging bag subjected to the one-packaging processing using the imaging data acquired by using the mobile terminal imaging data acquisition unit,
wherein the one-packaging medicine audit apparatus outputs dose information on medicines actually subjected to the one-packaging processing, by using the one-packaging processing apparatus as the one-packaging medicine information by using the first output unit, and
wherein the mobile terminal apparatus acquires the dose information on the medicines actually subjected to the one-packaging processing by using the one-packaging processing apparatus as the one-packaging medicine information, by using the one-packaging medicine information acquisition unit, and displays an image represented by imaging data of medicines required to be dosed next time, by using the display unit,
wherein the mobile terminal apparatus further includes:
a mix-up determination possibility determination unit, which is capable of determining whether a determination of the presence or absence of a mix-up in the medicines to be one-packaged in the one-packaging bags is possible based on mix-up possibility information, and
a replenishment medicine specification unit that is capable of specifying a type of a medicine required to be replenished using the one-packaging medicine information acquired by using the one-packaging medicine information acquisition unit and dose history information representing actually dosed medicines, and capable of displaying the type of the medicine required to be replenished specified by using the replenishment medicine specification unit, by using the display unit.

2. The one-packaging medicine audit system according to claim 1,
wherein the mobile terminal apparatus further includes an application storage unit that stores a medicine notebook application and an application execution unit that executes the medicine notebook application.

3. The one-packaging medicine audit system according to claim 1 or 2,
wherein the first output unit encodes the one-packaging medicine information and outputs the encoded information.

4. The one-packaging medicine audit system according to any one of claims 1 to 3,
wherein the first output unit outputs the one-packaging medicine information for medicines of which an audit determination is passed.

5. The one-packaging medicine audit system according to any one of claims 1 to 4,
wherein the first output unit outputs identification information on the medicines actually one-packaged by the one-packaging processing apparatus as the one-packaging medicine information.

6. The one-packaging medicine audit system according to any one of claims 1 to 5,
wherein the mobile terminal apparatus displays at least any one of an image represented by imaging data of medicines dosed in the past or an image represented by imaging data of medicines required to be dosed in the future, by using the display unit.

7. The one-packaging medicine audit system according to any one of claims 1 to 6,
wherein the mobile terminal apparatus enlarges and displays the image represented by the imaging data of the medicines required to be dosed next time, by using the display unit.

8. The one-packaging medicine audit system according to any one of claims 1 to 7,
wherein the mobile terminal apparatus highlights and displays information relating to a dose timing of the medicines required to be dosed next time with respect to the image represented by the imaging data of the medicines required to be dosed next time, by using the display unit.

9. The one-packaging medicine audit system according to any one of claims 1 to 8,
wherein the mobile terminal apparatus displays information for urging the dose according to an elapsed period from the dose timing, by using the display unit.

10. A one-packaging medicine information outputting method in a one-packaging medicine audit system comprising a one-packaging medicine audit apparatus, a mobile terminal apparatus, and an imaging data storage apparatus, the one-packaging medicine audit apparatus including a fist output unit, an imaging data acquisition unit and a second output unit, and the mobile terminal apparatus including a one-packaging medicine information acquisition unit, a mobile terminal imaging data acquisition unit and a display unit, the one-packaging medicine audit method comprising:
a step of outputting, by the one-packaging medicine audit apparatus using the first output unit, identification information on the medicines actually one-packaged by a one-packaging processing apparatus that performs one-packaging processing of the medicines based on prescription information, as one-packaging medicine information having a format capable of being acquired by a mobile terminal apparatus;
a step of acquiring by the one-packaging medicine audit apparatus using the imaging data acquisition unit, imaging data of medicines actually to be subjected to the one-packaging processing by using the one-packaging processing apparatus;
a step of outputting by the one-packaging medicine audit apparatus using the second output unit, the imaging data and the identification information on the medicines actually subjected to the one-packaging processing in correlation with each other;
a step of storing by the imaging data storage apparatus, the imaging data output from the one-packaging medicine audit apparatus;
a step of outputting by the one-packaging medicine audit apparatus using the first output unit, outputs dose information on medicines actually subjected to the one-packaging processing, by using the one-packaging processing apparatus as the one-packaging medicine information,
a step of acquiring by the mobile terminal apparatus using the one-packaging medicine information acquisition unit, the dose information on the medicines actually subjected to the one-packaging processing;
a step of acquiring by the mobile terminal apparatus using the mobile terminal imaging data acquisition unit, the imaging data correlated with the one-packaging medicine information from the imaging data storage apparatus, by using the acquired one-packaging medicine information;
a step of displaying by the mobile terminal apparatus using the display unit, an image represented by imaging data of medicines required to be dosed next time
a step of determining whether a determination of the presence or absence of a mix-up in the medicines to be one-packaged in the one-packaging bags is possible based on mix-up possibility information,
a step of specifying a type of a medicine required to be replenished using the one-packaging medicine information acquired by using the one-packaging medicine information acquisition unit and dose history information representing actually dosed medicines, and
a step of displaying the type of the medicine required to be replenished specified by using the replenishment medicine specification unit, by using the display unit.

11. A non-transitory comptuter-readable recording medium, recording a program for causing one or more computers to execute the one-packaging medicine information outputting method according to claim 10 in a case where the program is read.

## Patentansprüche

1. Prüfsystem für Arzneimittel in Einzelverpackung, umfassend eine Prüfvorrichtung für Arzneimittel in Einzelverpackung, eine mobile Terminalvorrichtung und eine Bilddaten-Speichervorrichtung, die Bilddaten für die Ausgabe aus der Prüfvorrichtung für Arzneimittel in Einzelverpackung speichert,
wobei die Prüfvorrichtung für Arzneimittel in Einzelverpackung umfasst:
eine erste Ausgabeeinheit, die Kennungsinformation ausgibt über Arzneimittel, die aktuell von einer Verarbeitungsvorrichtung für Einzelverpackung einzeln verpackt wurden, welche Vorrichtung eine Einzelverpackungsverarbeitung der Arzneimittel anhand von Rezeptinformation ausführt und die Kennungsinformation als Information über Arzneimittel in Einzelverpackung in einem Format ausgibt, welches von einem mobilen Terminal erfassbar ist;
eine Bilddaten-Erfassungseinheit, die Bilddaten von Arzneimitteln erfasst, die aktuell der Einzelverpackungsverarbeitung unterzogen werden, wozu die Verarbeitungsvorrichtung für Einzelverpackung verwendet wird; und
eine zweite Ausgabeeinheit, welche die unter Verwendung der Bilddaten-Erfassungseinheit erfassten Bilddaten und die Kennungsinformation bezüglich der aktuell der Einzelverpackungsverarbeitung unterzogenen Arzneimittel ausgibt unter Verwendung der Vorrichtung für Einzelverpackungsverarbeitung in Korrelation miteinander,
wobei das mobile Terminal umfasst:
eine Informationserfassungseinheit für Arzneimittel in Einzelverpackung, welche die von der Prüfvorrichtung für Arzneimittel in Einzelverpackung ausgegebene Information über Arzneimittel in Einzelverpackung erfasst;
eine Mobilterminal-Bilddatenerfassungseinheit, welche die Bilddaten korreliert mit der Information über Arzneimittel in Einzelverpackung aus der Bilddaten-Speichervorrichtung unter Verwendung der Information über Arzneimittel in Einzelverpackung, die mit Hilfe der Informationserfassungseinheit für Arzneimittel in Einzelverpackung erfasst wurde, erfasst; und
eine Anzeigeeinheit, die ein Bild für jeden Einzelverpackungsbeutel anzeigt, welcher der Einzelverpackungsverarbeitung unterworfen wird, unter Verwendung der Bilddaten, die mit Hilfe der Mobilterminal-Bilddatenerfassungseinheit erfasst wurden,
wobei die Prüfvorrichtung für Arzneimittel in Einzelverpackung Dosisinformation für Arzneimittel ausgibt, die aktuell der Einzelverpackungsverarbeitung unterzogen werden, wozu die Vorrichtung für Einzelverpackungsverarbeitung verwendet wird, als die Information über Arzneimittel-Einzelverpackung unter Verwendung der ersten Ausgabeeinheit, und
wobei das Mobilterminal die Dosisinformation über Arzneimittel erfasst, die aktuell der Einzelverpackungsverarbeitung unter Verwendung der Vorrichtung für Einzelverpackungsverarbeitung unterzogen werden, als die Information über Arzneimittel-Einzelverpackung, unter Verwendung der Informationserfassungseinheit für Arzneimittel-Einzelverpackung, und über die Anzeigeeinheit ein Bild zur Anzeige bringt, welches repräsentiert wird durch Bilddaten von Arzneimitteln, die beim nächsten Mal zu dosieren sind,
wobei das Mobilterminal weiterhin enthält:
eine Verwechslungsgefahr-Bestimmungseinheit, die in der Lage ist festzustellen, ob eine Bestimmung des Vorhandenseins oder Fehlens einer Verwechslung bei den in den Einzelverpackungsbeuteln enthaltenen einzeln verpackten Arzneimitteln möglich ist, basierend auf Verwechslungsgefahrinformation, und
eine Auffüll-Arzneimittelspezifikationseinheit, die in der Lage ist, einen Typ eines Arzneimittels zu spezifizieren, der aufgefüllt werden muss, unter Verwendung der Information über Arzneimittel-Einzelverpackung, die von der Informationserfassungseinheit für Arzneimittel in Einzelverpackung erfasst wurde, und unter Verwendung von Dosis-Vorgeschichtsinformation, die tatsächlich dosierte Arzneimittel repräsentiert, und die in der Lage ist, den Typ des Medikaments anzuzeigen, der aufzufüllen ist, spezifiziert unter Verwendung der Auffüll-Arzneimittelspezifikationseinheit, wozu die Anzeigeeinheit verwendet wird.

2. System nach Anspruch 1,
bei dem das Mobilterminal weiterhin eine Anwendungsspeichereinheit enthält, die eine Arzneimittel-Notebookanwendung und eine Anwendungs-Ausführeinheit, welche die Arzneimittel-Notebookanwendung ausführt, speichert.

3. System nach Anspruch 1 oder 2,
bei dem die erste Ausgabeeinheit die Information über Arzneimittel in Einzelverpackung kodiert und die kodierte Information ausgibt.

4. System nach einem der Ansprüche 1 bis 3,
bei dem die erste Ausgabeeinheit die Information über Arzneimittel in Einzelverpackung für solche Arzneimittel ausgibt, die die Prüffeststellung erfüllt haben.

5. System nach einem der Ansprüche 1 bis 4,
bei dem die erste Ausgabeeinheit Kennungsinformation über Arzneimittel ausgibt, die von der Vorrichtung für Einzelverpackungsverarbeitung tatsächlich einzeln verpackt wurden, als die Information über Arzneimittel in Einzelverpackung.

6. System nach einem der Ansprüche 1 bis 5,
bei dem das Mobilterminal mit Hilfe der Anzeigeeinheit ein Bild, welches durch Bilddaten von in der Vergangenheit dosierten Arzneimitteln repräsentiert, und/oder ein Bild anzeigt, welches durch Bilddaten von Arzneimitteln repräsentiert wird, die in Zukunft zu dosieren sind.

7. System nach einem der Ansprüche 1 bis 6,
bei dem das Mobilterminal das durch die Bilddaten von Arzneimitteln, die als nächstes zu dosieren sind, mit Hilfe der Anzeigeeinheit vergrößert und anzeigt.

8. System nach einem der Ansprüche 1 bis 7,
bei dem das Mobilterminal Information bezüglich einer Dosierzeit von Arzneimitteln, die als nächstes zu dosieren sind, in Bezug auf das Bild mit Hilfe der Anzeigeeinheit hervorhebt und anzeigt, das durch die Bilddaten von Arzneimitteln, die als nächstes zu dosieren sind, repräsentiert wird.

9. System nach einem der Ansprüche 1 bis 8,
bei dem das Mobilterminal unter Verwendung der Anzeigeeinheit Information anzeigt zum Erzwingen der Dosis nach Maßgabe einer nach der Dosierzeit verstrichenen Zeitspanne.

10. Einzelverpackungsarzneimittel-Informationsausgabeverfahren in einem Einzelverpackungs-Arzneimittel-Prüfsystem, welches eine Einzelverpackungsarzneimittel-Prüfvorrichtung, ein Mobilterminal und eine Bilddaten-Speichervorrichtung enthält, wobei die Einzelverpackungsarzneimittel-Prüfvorrichtung eine erste Ausgabeeinheit, eine Bilddaten-Erfassungseinheit und eine zweite Ausgabeeinheit enthält, und das Mobilterminal eine Einzelverpackungsarzneimittel-Informationserfassungseinheit, eine Mobilterminal-Bilddatenerfassungseinheit und eine Anzeigeeinheit enthält, wobei das Einzelverpackungsarzneimittel-Prüfverfahren umfasst:
einen Schritt, bei dem von der Einzelverpackungsarzneimittel-Prüfvorrichtung unter Verwendung der ersten Ausgabeeinheit Kennungsinformation über die Arzneimittel ausgegeben wird, die aktuell von einer Einzelverpackungs-Verarbeitungsvorrichtung verpackt werden, welche eine Einzelverpackungsverarbeitung von Arzneimitteln basierend auf Rezeptinformation ausführt, wobei die Ausgabe als Einzelverpackungs-Arzneimittelinformation mit einem Format ausgegeben wird, das von einem Mobilterminal erfasst werden kann;
einen Schritt, der durch die Einzelverpackungsarzneimittel-Prüfvorrichtung mit Hilfe der Bilddatenerfassungseinheit Bilddaten von Arzneimitteln erfasst, welche aktuell der Einzelverpackungsverarbeitung unter Verwendung der Einzelverpackungs-Verarbeitungsvorrichtung unterzogen werden;
einen Schritt, bei dem von der Einzelverpackungsarzneimittel-Prüfvorrichtung unter Verwendung der zweiten Ausgabeeinheit die Bilddaten und die Kennungsinformation über die Arzneimittel, die aktuell der Einzelverpackungsverarbeitung unterzogen werden, in Korrelation miteinander ausgegeben werden;
einen Schritt des Speicherns der von der Einzelverpackungsarzneimittel-Prüfvorrichtung ausgegebenen Bilddaten durch die Bilddaten-Speichervorrichtung;
einen Schritt, bei dem von der Einzelverpackungsarzneimittel-Prüfvorrichtung mit Hilfe der ersten Ausgabeeinheit Dosisinformation als die Einzelverpackungs-Arzneimittelinformation von Arzneimitteln ausgegeben werden, die aktuell von der Einzelverpackungs-Verarbeitungsvorrichtung der Einzelverpackungsverarbeitung unterzogen werden,
einen Schritt, der unter Verwendung der Einzelverpackungsarzneimittel-Informationserfassungseinheit durch das Mobilterminal die Dosisinformation über die Arzneimittel erfasst, die aktuell der Einzelverpackungsverarbeitung unterzogen werden;
einen Schritt, der durch das Mobilterminal unter Verwendung der Mobilterminal-Bilddatenerfassungseinheit die Bilddaten korreliert mit der Einzelverpackungsarzneimittel-Information aus der Bilddaten-Speichervorrichtung unter Verwendung der erfassten Einzelverpackungsarzneimittel-Information erfasst;
einen Schritt des Anzeigens durch das Mobilterminal unter Verwendung der Anzeigeeinheit eines Bilds, das repräsentiert wird durch Bilddaten von Arzneimitteln, die als nächstes zu dosieren sind;
einen Schritt des Bestimmens, ob eine Bestimmung des Vorhandenseins oder Fehlens einer Verwechslung unter den in Einzelverpackungsbeuteln einzeln zu verpackenden Arzneimitteln möglich ist, basierend auf Verwechslungsgefahrinformation,
einen Schritt des Spezifizierens eines aufzufüllenden Medikaments unter Verwendung der Einzelverpackungsarzneimittel-Information, die von der Einzelverpackungsarzneimittel-Informationserfassungseinheit erfasst wurde, und unter Verwendung von Dosisvorgeschichtsinformation, repräsentiert durch aktuell dosierte Arzneimittel, und
einen Schritt, bei dem über die Anzeigeeinheit der Typ des aufzufüllenden Medikaments angezeigt wird, welches unter Verwendung der Auffüllmedikament-Spezifikationseinheit spezifiziert wird.

11. Nicht flüchtiges, computerlesbares Aufzeichnungsmedium, welches ein Programm aufzeichnet, das ein oder mehrere Computer veranlasst, das Einzelverpackungsarzneimittel-Informationsausgabeverfahren nach Anspruch 10 auszuführen, falls das Programm gelesen wird.

## Revendications

1. Système d'audit de médicament sous conditionnement unitaire, comprenant un appareil d'audit de médicament sous conditionnement unitaire, un appareil terminal mobile, et un appareil de stockage de données d'imagerie, lequel stocke des données d'imagerie devant être produites à partir de l'appareil d'audit de médicament sous conditionnement unitaire,
dans lequel l'appareil d'audit de médicament sous conditionnement unitaire comprend :
une première unité de sortie, laquelle produit des informations d'identification sur les médicaments réellement sous conditionnement unitaire par un appareil de traitement de conditionnement unitaire, lequel réalise un traitement de conditionnement unitaire des médicaments sur la base des informations de prescription, comme informations de médicament sous conditionnement unitaire présentant un format pouvant être acquis par un appareil terminal mobile ;
une unité d'acquisition de données d'imagerie, laquelle acquiert des données d'imagerie de médicaments devant être soumis au traitement de conditionnement unitaire à l'aide de l'appareil de traitement de conditionnement unitaire, et
une seconde unité de sortie, laquelle produit les données d'imagerie acquises à l'aide de l'unité d'acquisition de données d'imagerie, et les données d'identification sur les médicaments réellement soumis au traitement de conditionnement unitaire à l'aide de l'appareil de traitement de conditionnement unitaire, en corrélation les unes avec les autres,
dans lequel l'appareil de terminal mobile comprend :
une unité d'acquisition d'informations de médicament sous conditionnement unitaire, laquelle acquiert les informations de médicament sous conditionnement unitaire produites à partir de l'appareil d'audit de médicament sous conditionnement unitaire ;
une unité d'acquisition de données d'imagerie de terminal mobile, laquelle acquiert les données d'imagerie corrélées avec les informations de médicament sous conditionnement unitaire à partir de l'appareil de stockage de données d'imagerie, à l'aide des informations de médicament sous conditionnement unitaire acquises à l'aide de l'unité d'acquisition d'informations de médicament sous conditionnement unitaire, et
une unité d'affichage, laquelle affiche une image pour chaque sachet de conditionnement unitaire soumis au traitement de conditionnement unitaire, à l'aide des données d'imagerie acquises à l'aide de l'unité d'acquisition de données d'imagerie de terminal mobile ;
dans lequel l'appareil d'audit de médicament sous conditionnement unitaire produit des informations de dose quant aux médicaments réellement soumis au traitement de conditionnement unitaire, à l'aide de l'appareil de traitement de conditionnement unitaire comme informations de médicament sous conditionnement unitaire à l'aide de la première unité de sortie, et
dans lequel l'appareil de terminal mobile acquiert les informations de dose sur les médicaments réellement soumis au traitement de conditionnement unitaire à l'aide de l'appareil de traitement de conditionnement unitaire comme informations de médicament sous conditionnement unitaire, à l'aide de l'unité d'acquisition d'informations de médicament sous conditionnement unitaire, et affiche une image représentée en imageant des données de médicaments devant être dosés une prochaine fois, à l'aide de l'unité d'affichage, et
dans lequel l'appareil de terminal mobile inclut en outre :
une unité de détermination de possibilité de détermination de méprise, laquelle est en mesure de déterminer si oui ou non une détermination de la présence ou de l'absence de méprise quant aux médicaments devant subir un conditionnement unitaire dans les sachets de conditionnement unitaire est possible, sur la base d'informations de possibilité de méprise, et
une unité de spécification de médicament pour réapprovisionnement, laquelle est en mesure de spécifier un type de médicament devant être réapprovisionné à l'aide des informations de médicament sous conditionnement unitaire acquises à l'aide de l'unité d'acquisition d'informations de médicament sous conditionnement unitaire et d'informations d'historique de dose représentant des médicament réellement dosés, et en mesure d'afficher le type de médicament devant être réapprovisionné spécifié à l'aide de l'unité de spécification de médicament pour réapprovisionnement, à l'aide de l'unité d'affichage.

2. Système d'audit de médicament sous conditionnement unitaire selon la revendication 1,
dans lequel l'appareil de terminal mobile inclut en outre une unité de stockage d'application, laquelle stocke une application bloc-notes de médicament et une unité d'exécution d'application, laquelle exécute l'application bloc-notes de médicament.

3. Système d'audit de médicament sous conditionnement unitaire selon la revendication 1 ou 2,
dans lequel la première unité de sortie code les informations de médicament sous conditionnement unitaire, et produit les informations codées.

4. Système d'audit de médicament sous conditionnement unitaire selon l'une quelconque des revendications 1 à 3,
dans lequel la première unité de sortie produit les informations de médicament sous conditionnement unitaire pour les médicaments pour lesquels une détermination d'audit est réussie.

5. Système d'audit de médicament sous conditionnement unitaire selon l'une quelconque des revendications 1 à 4,
dans lequel la première unité de sortie produit des informations d'identification sur les médicaments réellement soumis au conditionnement unitaire par l'appareil de traitement de conditionnement unitaire comme informations de médicament sous conditionnement unitaire.

6. Système d'audit de médicament sous conditionnement unitaire selon l'une quelconque des revendications 1 à 5,
dans lequel l'appareil de terminal mobile affiche au moins une image quelconque parmi une image représentée par des données d'imagerie de médicaments dosés dans le passé ou une image représentée par des données d'imagerie de médicaments devant être dosés à l'avenir, à l'aide de l'unité d'affichage.

7. Système d'audit de médicament sous conditionnement unitaire selon l'une quelconque des revendications 1 à 6,
dans lequel l'appareil de terminal mobile agrandit et affiche l'image représentée par les données d'imagerie des médicaments devant être dosés une prochaine fois, à l'aide de l'unité d'affichage.

8. Système d'audit de médicament sous conditionnement unitaire selon l'une quelconque des revendications 1 à 7,
dans lequel l'appareil de terminal mobile met en valeur et affiche des informations liées à une heure de dose des médicaments devant être dosés une prochaine fois par rapport à l'image représentée par les données d'imagerie des médicaments devant être dosés une prochaine fois, à l'aide de l'unité d'affichage.

9. Système d'audit de médicament sous conditionnement unitaire selon l'une quelconque des revendications 1 à 8,
dans lequel l'appareil de terminal mobile affiche des informations pour préconiser la dose conformément à une période écoulée depuis l'heure de dose, à l'aide de l'unité d'affichage.

10. Procédé de production d'informations de médicament sous conditionnement unitaire dans un système d'audit de médicament sous conditionnement unitaire comprenant un appareil d'audit de médicament sous conditionnement unitaire, un appareil terminal mobile, et un appareil de stockage de données d'imagerie, l'appareil d'audit de médicament sous conditionnement unitaire incluant une première unité de sortie, une unité d'acquisition de données d'imagerie, et une seconde unité de sortie, et l'appareil terminal mobile incluant une unité d'acquisition de médicaments sous conditionnement unitaire, une unité d'acquisition de données d'imagerie de terminal mobile, et une unité d'affichage, le procédé d'audit d'informations de médicament sous conditionnement unitaire comprenant les étapes suivantes :
une étape de production, par l'appareil d'audit de médicament sous conditionnement unitaire à l'aide de la première unité de sortie, d'informations d'identification sur les médicaments réellement sous conditionnement unitaire par un appareil de traitement de conditionnement unitaire, lequel réalise un traitement de conditionnement unitaire des médicaments sur la base des informations de prescription, comme informations de médicament sous conditionnement unitaire présentant un format pouvant être acquis par un appareil terminal mobile ;
une étape d'acquisition, par l'appareil d'audit de médicament sous conditionnement unitaire à l'aide de l'unité d'acquisition de données d'imagerie, de données d'imagerie de médicaments devant être soumis au traitement de conditionnement unitaire à l'aide de l'appareil de traitement de conditionnement unitaire, et
une étape de production, par l'appareil d'audit de médicament sous conditionnement unitaire à l'aide de la seconde unité de sortie, de données d'imagerie et de données d'identification sur les médicaments réellement soumis au traitement de conditionnement unitaire, en corrélation les unes avec les autres ;
une étape de stockage, par l'appareil de stockage de données d'imagerie, des données d'imagerie produites à partir de l'appareil d'audit de médicament sous conditionnement unitaire ;
une étape de production, par l'appareil d'audit de médicament sous conditionnement unitaire à l'aide de la première unité de sortie, pour produire des informations de dose sur des médicaments réellement soumis au traitement de conditionnement unitaire, à l'aide de l'appareil de traitement de conditionnement unitaire comme informations de médicament sous conditionnement unitaire ;
une étape d'acquisition, par l'appareil de terminal mobile à l'aide de l'unité d'acquisition d'informations de médicament sous conditionnement unitaire, des informations de dose sur les médicaments réellement soumis au traitement de conditionnement unitaire ;
une étape d'acquisition par l'appareil de terminal mobile à l'aide de l'unité d'acquisition de données d'imagerie de terminal mobile, des données d'imagerie corrélées avec les informations de médicament sous conditionnement unitaire à partir de l'appareil de stockage de données d'imagerie, à l'aide des informations de médicament sous conditionnement unitaire acquises ;
une étape d'affichage par l'appareil de terminal mobile à l'aide de l'unité d'affichage, d'une image représentée par les données d'imagerie de médicaments devant être dosés une prochaine fois ;
une étape de détermination si oui ou non une détermination de la présence ou de l'absence de méprise quant aux médicaments sous conditionnement unitaire dans les sachets de conditionnement unitaire est possible, sur la base d'informations de possibilité de méprise, et
une étape de spécification de type de médicament devant être réapprovisionné à l'aide des informations de médicament sous conditionnement unitaire acquises à l'aide de l'unité d'acquisition d'informations de médicament sous conditionnement unitaire et d'informations d'historique de dose représentant des médicaments réellement dosés, et
une étape d'affichage du type de médicament devant être réapprovisionné spécifié à l'aide de l'unité de spécification de médicament pour réapprovisionnement, à l'aide de l'unité d'affichage.

11. Support d'enregistrement lisible par ordinateur non transitoire, enregistrant un programme pour faire en sorte qu'un ou plusieurs ordinateurs exécutent le procédé de production d'informations de médicament sous conditionnement unitaire selon la revendication 10 dans le cas où le programme est lu.
